# EUROPEAN PATENT APPLICATION

(11) **EP 4 796 638 A1**
(43) Date of publication of application: **26.08.2026**
(21) Application number: 24879810.0
(22) Date of filing: 18.10.2024
(51) Int. Cl.: C12N 15/31, A01H 5/00, A01H 5/02, A01H 6/14, A01H 6/30, A01H 6/56, A01H 6/74, A23L 5/43, C07H 19/044, C12N 5/04, C12N 5/10, C12N 15/53, C12N 15/63, C12N 15/82

(54) **TRANSGENIC PLANT WITH ALTERED FLOWER COLORATION, AND NOVEL COMPOUND CONTAINED IN SAME**

(30) Priority: 20.10.2023 JP 2023181380
(71) Applicant: Suntory Holdings Limited, Osaka-shi, Osaka 530-8203 (JP)
(72) Inventor: NAKAMURA, Noriko, Soraku-gun, Kyoto 619-0284 (JP); KATSUMOTO, Yukihisa, Soraku-gun, Kyoto 619-0284 (JP); SUGAHARA, Kohtaro, Soraku-gun, Kyoto 619-0284 (JP)
(74) Representative: Vossius & Partner Patentanwälte Rechtsanwälte mbB
(86) International application number: PCT/JP2024/037130
(87) International publication number: WO 2025/084390

(57) **Abstract**

The present invention provides: a transgenic plant having a blue flower color; a method for creating same; a novel compound contained in the transgenic plant; and a method for producing same. In the present invention, a VioA gene, a VioB gene, a VioC gene, a VioE gene or a combination of these is introduced into cells of a plant containing L-tryptophan.

## Description

### Technical Field

The present invention relates to a transgenic plant with alteration of flower color; a method for creating the same; a novel compound contained in the transgenic plant; and a method for producing the same.

### Background Art

Rose, chrysanthemum, and carnation are industrially important ornamental flowers worldwide. In particular, rose, being the most popular ornamental plant with a record of cultivation since BC, has been artificially crossbred for hundreds of years. However, issues such as the absence of wild varieties with blue flower color among hybridizable related species have made it difficult to create rose varieties with blue flower color by known cross-breeding and mutation breeding. The creation of a completely new blue flower color helps arouse new demand for even wider uses of ornamental flowers and contributes to increased production and consumption. Therefore, attempts have been made to produce roses with blue flower colors by genetic engineering techniques.

Since flowers in plants in nature express vivid blue color through various mechanisms such as a copigment effect (intermolecular association), polyacylation (intramolecular association), formation of a metal complex, and an increase in vacuolar pH, a rose variety having blue flower color has been developed by applying such a mechanism of blue flower color to a rose, but it has been considered that application of the color development mechanism in organisms other than plants to alter flower color is extremely difficult.

Gram-negative bacteria such as Chromobacterium violaceum are known to biosynthesize violacein and deoxyviolacein as natural blue pigments.

Patent Document 1 discloses that violacein can be used as a natural blue dye.

Patent Document 2 discloses a treatment method for improving light resistance of a fiber and textile product dyed with violacein or deoxyviolacein.

Patent Document 3 discloses a method for producing deoxyviolacein by knocking out VioD in a violacein synthesis-related gene cluster including VioA, VioB, VioC, VioD, and VioE.

Patent Document 4 discloses a method for efficiently producing violacein or deoxyviolacein by using a microorganism in which the activity of a protein having VioA activity, VioB activity, VioC activity, VioD activity, and VioE activity is enhanced.

However, there has hardly been known an attempt to alter flower color by accumulating violaceins originally produced by microorganisms in petals of plants.

### Citation List

### Patent Document

Patent Document 1: JP H06-253864 A
Patent Document 2: JP H11-152687 A
Patent Document 3: JP 2011-527183 T
Patent Document 4: JP 2021-19518 A

### Non-Patent Document

Non-Patent Document 1: Transient gene expression in rose petals via Agrobacterium Infiltration, Plant Cell Tiss Organ Cult (2010) 102: 245-250

### Summary of Invention

### Technical Problem

The issue to be solved by the present invention is to provide a transgenic plant or its inbred or outbred progeny, or a propagule, partial plant body, tissue, or cell thereof, with alteration of flower color, preferably alteration to a blue flower color, and to provide a novel compound contained in the transgenic plant.

### Solution to Problem

As a result of intensive studies, surprisingly, the present inventors have succeeded in accumulating, as blue pigments, deoxyviolacein and an unknown compound in cells of a plant by modifying four genes, VioA, VioB, VioC, and VioE, which are required for synthesis of deoxyviolacein using L-tryptophan as a starting substrate, to introduce the modified genes into the cells. Furthermore, the present inventors have isolated and purified a blue pigment of the unknown compound accumulated in the cells of the plant, in addition to deoxyviolacein, and have succeeded in identifying the compound as deoxyviolacein 15N-β-D-glucopyranoside, thus completed the present invention.

The present invention relates to as follows.
[1] A VioA gene selected from the group consisting of:
   (1-a) a polynucleotide consisting of a nucleotide sequence of SEQ ID NO: 1;
   (1-b) a polynucleotide that hybridizes under stringent conditions with another polynucleotide consisting of a nucleotide sequence complementary to the nucleotide sequence of SEQ ID NO: 1, wherein the polynucleotide encodes a protein having a function of converting L-tryptophan into indole-3-pyruvic acid imine (IPA imine);
   (1-c) a polynucleotide that encodes a protein consisting of an amino acid sequence of SEQ ID NO: 2;
   (1-d) a polynucleotide that encodes a protein consisting of an amino acid sequence in which one or several amino acids are deleted, substituted, inserted, and/or added in the amino acid sequence of SEQ ID NO: 2, the protein having a function of converting L-tryptophan into indole-3-pyruvic acid imine (IPA imine); and
   (1-e) a polynucleotide that encodes a protein having an amino acid sequence with 90% or more identity to the amino acid sequence of SEQ ID NO: 2, the protein having a function of converting L-tryptophan into indole-3-pyruvic acid imine (IPA imine).
[2] A VioB gene selected from the group consisting of:
   (2-a) a polynucleotide consisting of a nucleotide sequence of SEQ ID NO: 3;
   (2-b) a polynucleotide that hybridizes under stringent conditions with another polynucleotide consisting of nucleotide sequence complementary to the nucleotide sequence of SEQ ID NO: 3, wherein the polynucleotide encodes a protein having a function of converting IPA imine into an IPA imine dimer;
   (2-c) a polynucleotide that encodes a protein consisting of an amino acid sequence of SEQ ID NO: 4;
   (2-d) a polynucleotide that encodes a protein consisting of an amino acid sequence in which one or several amino acids are deleted, substituted, inserted, and/or added in the amino acid sequence of SEQ ID NO: 4, the protein having a function of converting IPA imine into an IPA imine dimer; and
   (2-e) a polynucleotide that encodes a protein having an amino acid sequence with 90% or more identity to the amino acid sequence of SEQ ID NO: 4, the protein having a function of converting IPA imine into an IPA imine dimer.
[3] A VioC gene selected from the group consisting of:
   (3-a) a polynucleotide consisting of a nucleotide sequence of SEQ ID NO: 5;
   (3-b) a polynucleotide that hybridizes under stringent conditions with another polynucleotide consisting of a nucleotide sequence complementary to the nucleotide sequence of SEQ ID NO: 5, wherein the polynucleotide encodes a protein having a function of converting prodeoxyviolaceinic acid into deoxyviolaceinic acid;
   (3-c) a polynucleotide that encodes a protein consisting of an amino acid sequence of SEQ ID NO: 6;
   (3-d) a polynucleotide that encodes a protein consisting of an amino acid sequence in which one or several amino acids are deleted, substituted, inserted, and/or added in the amino acid sequence of SEQ ID NO: 6, the protein having a function of converting prodeoxyviolaceinic acid into deoxyviolaceinic acid; and
   (3-e) a polynucleotide that encodes a protein having an amino acid sequence with 90% or more identity to the amino acid sequence of SEQ ID NO: 6, the protein having a function of converting prodeoxyviolaceinic acid into deoxyviolaceinic acid.
[4] A VioE gene selected from the group consisting of:
   (4-a) a polynucleotide consisting of a nucleotide sequence of SEQ ID NO: 7;
   (4-b) a polynucleotide that hybridizes under stringent conditions with another polynucleotide consisting of a nucleotide sequence complementary to the nucleotide sequence of SEQ ID NO: 7, wherein the polynucleotide encodes a protein having a function of converting an IPA imine dimer into prodeoxyviolaceinic acid;
   (4-c) a polynucleotide that encodes a protein consisting of an amino acid sequence of SEQ ID NO: 8;
   (4-d) a polynucleotide that encodes a protein consisting of an amino acid sequence in which one or several amino acids are deleted, substituted, inserted, and/or added in the amino acid sequence of SEQ ID NO: 8, the protein having a function of converting an IPA imine dimer into prodeoxyviolaceinic acid; and
   (4-e) a polynucleotide that encodes a protein having an amino acid sequence with 90% or more identity to the amino acid sequence of SEQ ID NO: 8, the protein having a function of converting an IPA imine dimer into prodeoxyviolaceinic acid.
[5] A vector, including a VioA gene, a VioB gene, a VioC gene, a VioE gene, or any combination thereof, wherein
   the VioA gene is selected from the group consisting of:
      (1-a) a polynucleotide consisting of a nucleotide sequence of SEQ ID NO: 1;
      (1-b) a polynucleotide that hybridizes under stringent conditions with another polynucleotide consisting of a nucleotide sequence complementary to the nucleotide sequence of SEQ ID NO: 1, wherein the polynucleotide encodes a protein having a function of converting L-tryptophan into indole-3-pyruvic acid imine (IPA imine);
      (1-c) a polynucleotide that encodes a protein consisting of an amino acid sequence of SEQ ID NO: 2;
      (1-d) a polynucleotide that encodes a protein consisting of an amino acid sequence in which one or several amino acids are deleted, substituted, inserted, and/or added in the amino acid sequence of SEQ ID NO: 2, the protein having a function of converting L-tryptophan into indole-3-pyruvic acid imine (IPA imine); and
      (1-e) a polynucleotide that encodes a protein having an amino acid sequence with 90% or more identity to the amino acid sequence of SEQ ID NO: 2, the protein having a function of converting L-tryptophan into indole-3-pyruvic acid imine (IPA imine),
   the VioB gene is selected from the group consisting of:
      (2-a) a polynucleotide consisting of a nucleotide sequence of SEQ ID NO: 3;
      (2-b) a polynucleotide that hybridizes under stringent conditions with another polynucleotide consisting of a nucleotide sequence complementary to the nucleotide sequence of SEQ ID NO: 3, wherein the polynucleotide encodes a protein having a function of converting IPA imine into an IPA imine dimer;
      (2-c) a polynucleotide that encodes a protein consisting of an amino acid sequence of SEQ ID NO: 4;
      (2-d) a polynucleotide that encodes a protein consisting of an amino acid sequence in which one or several amino acids are deleted, substituted, inserted, and/or added in the amino acid sequence of SEQ ID NO: 4, the protein having a function of converting IPA imine into an IPA imine dimer; and
      (2-e) a polynucleotide that encodes a protein having an amino acid sequence with 90% or more identity to the amino acid sequence of SEQ ID NO: 4, the protein having a function of converting IPA imine into an IPA imine dimer,
   the VioC gene is selected from the group consisting of:
      (3-a) a polynucleotide consisting of a nucleotide sequence of SEQ ID NO: 5;
      (3-b) a polynucleotide that hybridizes under stringent conditions with another polynucleotide consisting of a nucleotide sequence complementary to the nucleotide sequence of SEQ ID NO: 5, wherein the polynucleotide encodes a protein having a function of converting prodeoxyviolaceinic acid into deoxyviolaceinic acid;
      (3-c) a polynucleotide that encodes a protein consisting of an amino acid sequence of SEQ ID NO: 6;
      (3-d) a polynucleotide that encodes a protein consisting of an amino acid sequence in which one or several amino acids are deleted, substituted, inserted, and/or added in the amino acid sequence of SEQ ID NO: 6, the protein having a function of converting prodeoxyviolaceinic acid into deoxyviolaceinic acid; and
      (3-e) a polynucleotide that encodes a protein having an amino acid sequence with 90% or more identity to the amino acid sequence of SEQ ID NO: 6, the protein having a function of converting prodeoxyviolaceinic acid into deoxyviolaceinic acid, and
   the VioE gene is selected from the group consisting of:
      (4-a) a polynucleotide consisting of a nucleotide sequence of SEQ ID NO: 7;
      (4-b) a polynucleotide that hybridizes under stringent conditions with another polynucleotide consisting of a nucleotide sequence complementary to the nucleotide sequence of SEQ ID NO: 7, wherein the polynucleotide encodes a protein having a function of converting an IPA imine dimer into prodeoxyviolaceinic acid;
      (4-c) a polynucleotide that encodes a protein consisting of an amino acid sequence of SEQ ID NO: 8;
      (4-d) a polynucleotide that encodes a protein consisting of an amino acid sequence in which one or several amino acids are deleted, substituted, inserted, and/or added in the amino acid sequence of SEQ ID NO: 8, the protein having a function of converting an IPA imine dimer into prodeoxyviolaceinic acid; and
      (4-e) a polynucleotide that encodes a protein having an amino acid sequence with 90% or more identity to the amino acid sequence of SEQ ID NO: 8, the protein having a function of converting an IPA imine dimer into prodeoxyviolaceinic acid.
[6] The vector according to 5, further including the VioA gene and a promoter linked to the VioA gene and configured to function as a petal-specific promoter, wherein the promoter is selected from a lisianthus-derived anthocyanidin synthase gene promoter (SEQ ID NO: 9), a morning glory-derived dihydroflavonol 4-reductase gene promoter (SEQ ID NO: 10), or a rose-derived chalcone synthase gene promoter (SEQ ID NO: 11).
[7] The vector according to 5 or 6, further including an untranslated region (5'-UTR) derived from Arabidopsis thaliana-derived alcohol dehydrogenase (ADH) gene (SEQ ID NO: 12) operably linked to the VioA gene, the VioB gene, the VioC gene, and/or the VioE gene.
[8] A transgenic plant or its inbred or outbred progeny, including a VioA gene, a VioB gene, a VioC gene, a VioE gene, or any combination thereof, wherein
   the VioA gene is selected from the group consisting of:
      (1-a) a polynucleotide consisting of a nucleotide sequence of SEQ ID NO: 1;
      (1-b) a polynucleotide that hybridizes under stringent conditions with another polynucleotide consisting of a nucleotide sequence complementary to the nucleotide sequence of SEQ ID NO: 1, wherein the polynucleotide encodes a protein having a function of converting L-tryptophan into indole-3-pyruvic acid imine (IPA imine);
      (1-c) a polynucleotide that encodes a protein consisting of an amino acid sequence of SEQ ID NO: 2;
      (1-d) a polynucleotide that encodes a protein consisting of an amino acid sequence in which one or several amino acids are deleted, substituted, inserted, and/or added in the amino acid sequence of SEQ ID NO: 2, the protein having a function of converting L-tryptophan into indole-3-pyruvic acid imine (IPA imine); and
      (1-e) a polynucleotide that encodes a protein having an amino acid sequence with 90% or more identity to the amino acid sequence of SEQ ID NO: 2, the protein having a function of converting L-tryptophan into indole-3-pyruvic acid imine (IPA imine),
   the VioB gene is selected from the group consisting of:
      (2-a) a polynucleotide consisting of a nucleotide sequence of SEQ ID NO: 3;
      (2-b) a polynucleotide that hybridizes under stringent conditions with another polynucleotide consisting of a nucleotide sequence complementary to the nucleotide sequence of SEQ ID NO: 3, wherein the polynucleotide encodes a protein having a function of converting IPA imine into an IPA imine dimer;
      (2-c) a polynucleotide that encodes a protein consisting of an amino acid sequence of SEQ ID NO: 4;
      (2-d) a polynucleotide that encodes a protein consisting of an amino acid sequence in which one or several amino acids are deleted, substituted, inserted, and/or added in the amino acid sequence of SEQ ID NO: 4, the protein having a function of converting IPA imine into an IPA imine dimer; and
      (2-e) a polynucleotide that encodes a protein having an amino acid sequence with 90% or more identity to the amino acid sequence of SEQ ID NO: 4, the protein having a function of converting IPA imine into an IPA imine dimer,
   the VioC gene is selected from the group consisting of:
      (3-a) a polynucleotide consisting of a nucleotide sequence of SEQ ID NO: 5;
      (3-b) a polynucleotide that hybridizes under stringent conditions with another polynucleotide consisting of a nucleotide sequence complementary to the nucleotide sequence of SEQ ID NO: 5, wherein the polynucleotide encodes a protein having a function of converting prodeoxyviolaceinic acid into deoxyviolaceinic acid;
      (3-c) a polynucleotide that encodes a protein consisting of an amino acid sequence of SEQ ID NO: 6;
      (3-d) a polynucleotide that encodes a protein consisting of an amino acid sequence in which one or several amino acids are deleted, substituted, inserted, and/or added in the amino acid sequence of SEQ ID NO: 6, the protein having a function of converting prodeoxyviolaceinic acid into deoxyviolaceinic acid; and
      (3-e) a polynucleotide that encodes a protein having an amino acid sequence with 90% or more identity to the amino acid sequence of SEQ ID NO: 6, the protein having a function of converting prodeoxyviolaceinic acid into deoxyviolaceinic acid, and
   the VioE gene is selected from the group consisting of:
      (4-a) a polynucleotide consisting of a nucleotide sequence of SEQ ID NO: 7;
      (4-b) a polynucleotide that hybridizes under stringent conditions with another polynucleotide consisting of a nucleotide sequence complementary to the nucleotide sequence of SEQ ID NO: 7, wherein the polynucleotide encodes a protein having a function of converting an IPA imine dimer into prodeoxyviolaceinic acid;
      (4-c) a polynucleotide that encodes a protein consisting of an amino acid sequence of SEQ ID NO: 8;
      (4-d) a polynucleotide that encodes a protein consisting of an amino acid sequence in which one or several amino acids are deleted, substituted, inserted, and/or added in the amino acid sequence of SEQ ID NO: 8, the protein having a function of converting an IPA imine dimer into prodeoxyviolaceinic acid; and
      (4-e) a polynucleotide that encodes a protein having an amino acid sequence with 90% or more identity to the amino acid sequence of SEQ ID NO: 8, the protein having a function of converting an IPA imine dimer into prodeoxyviolaceinic acid.
[9] The transgenic plant or its inbred or outbred progeny according to 8, wherein the plant is a rose, carnation, chrysanthemum, or lily.
[10] A propagule, partial plant body, tissue, or cell of the transgenic plant or its inbred or outbred progeny according to 8 or 9.
[11] A cut flower of the transgenic plant or its inbred or outbred progeny according to 8 or 9, or a processed form created from the cut flower.
[12] A method for producing a transgenic plant, the method, including introducing the gene according to any of 1 to 4 or the vector according to any of 5 to 7 into a cell of a plant containing L-tryptophan.
[13] The method according to 12, wherein the plant is a rose, carnation, chrysanthemum, or lily.
[14] A compound represented by a formula set forth below: 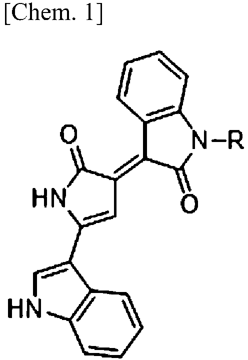 where in the formula, R is a monosaccharide unit selected from glucose, mannose, galactose, fucose, rhamnose, arabinose, xylose, fructose, or glucuronic acid, or a sugar chain formed by linking two or more of the monosaccharide units.
[15] The compound according to 14, wherein R in the formula is glucose.
[16] A dye, including the compound according to 14 or 15.
[17] A textile product, including the dye according to 16.
[18] A colorant, including the compound according to 14 or 15.
[19] A medicament, cosmetic, food, or beverage product, including the compound according to 14 or 15.
[20] A method for producing deoxyviolacein and/or a glycoside thereof, the method including:
   introducing the gene according to any of 1 to 4 or the vector according to any of 5 to 7 into a cell of a plant containing L-tryptophan; and
   collecting the compound from the cell.
[21] The method according to 20, wherein the glycoside is the compound according to 14 or 15.
[22] The method according to 20 or 21, wherein the plant is a rose, carnation, chrysanthemum, or lily.
[23] A plant or its inbred or outbred progeny, including deoxyviolacein and/or a glycoside thereof.
[24] The plant or its inbred or outbred progeny according to 23, wherein the glycoside is the compound according to 14 or 15.
[25] The plant or its inbred or outbred progeny according to any of 23 and 24, wherein the plant is a rose, carnation, chrysanthemum, or lily.
[26] A propagule, partial plant body, tissue, or cell of the plant or its inbred or outbred progeny according to any of 23 to 25.
[27] A cut flower of the plant or its inbred or outbred progeny according to any of 23 to 25, or a processed form created from the cut flower.

### Advantageous Effects of Invention

According to the present invention, it is possible to provide a transgenic plant or its inbred or outbred progeny, or a propagule, partial plant body, tissue, or cell thereof, with alteration of flower color, preferably alteration to a blue flower color. According to the present invention, it is also possible to provide a novel compound, deoxyviolacein 15N-β-D-glucopyranoside, as a natural blue pigment. The compound can be used as a dye or a colorant.

### Brief Description of Drawings

FIG. 1 illustrates a biosynthetic pathway for violaceins using L-tryptophan as a starting substrate.
FIG. 2 is a construction diagram of a binary vector pSPB8555.
FIG. 3 presents a high-performance liquid chromatogram of petal extracts from roses (varieties: Ocean Song (OS), Be Sweet (BS), Nectarine (NT), Lavande (LA), Mondial (MD), Arleene (AL), and WKS82) into which pSPB8555 has been introduced.
FIG. 4 presents a high-performance liquid chromatogram of a petal extract from carnation (variety: Durcal (DC)) into which pSPB8555 has been introduced.
FIG. 5 is a construction diagram of a binary vector pSPB7673.
FIG. 6 is a construction diagram of a binary vector pSPB7674.
FIG. 7 is a construction diagram of a binary vector pSPB7677.
FIG. 8 presents a high-performance liquid chromatogram of a callus extract from rose (variety: Ocean Song) into which pSPB8555, pSPB7673, pSPB7674, or pSPB7677 has been introduced.
FIG. 9 presents a preparative HPLC chromatogram (570 nm) of a 60% methanol fraction containing a compound (I).
FIG. 10 is an ultraviolet-visible absorption spectrum of a compound (I).
FIG. 11-1 is ¹H-NMR spectrum of a compound (I).
FIG. 11-2 is a ¹³C-NMR spectrum of a compound (I).
FIG. 12 presents a preparative HPLC chromatogram (570 nm) of an 80% methanol fraction containing a compound (II).
FIG. 13 is an ultraviolet-visible absorption spectrum of a compound (II).
FIG. 14-1 is ¹H-NMR spectrum of a compound (II).
FIG. 14-2 is a ¹³C-NMR spectrum of a compound (II).

Violacein, a secondary metabolite synthesized by gram-negative bacteria such as Chromobacterium violaceum, is a natural blue-violet pigment. In addition, violacein has attracted attention for its functionality such as antitumor activity, antibacterial activity, antimicrobial activity, and antiviral activity. Violacein is produced through 5 steps of enzymatic reactions by VioA, VioB, VioC, VioD, and VioE using L-tryptophan as a starting substrate.

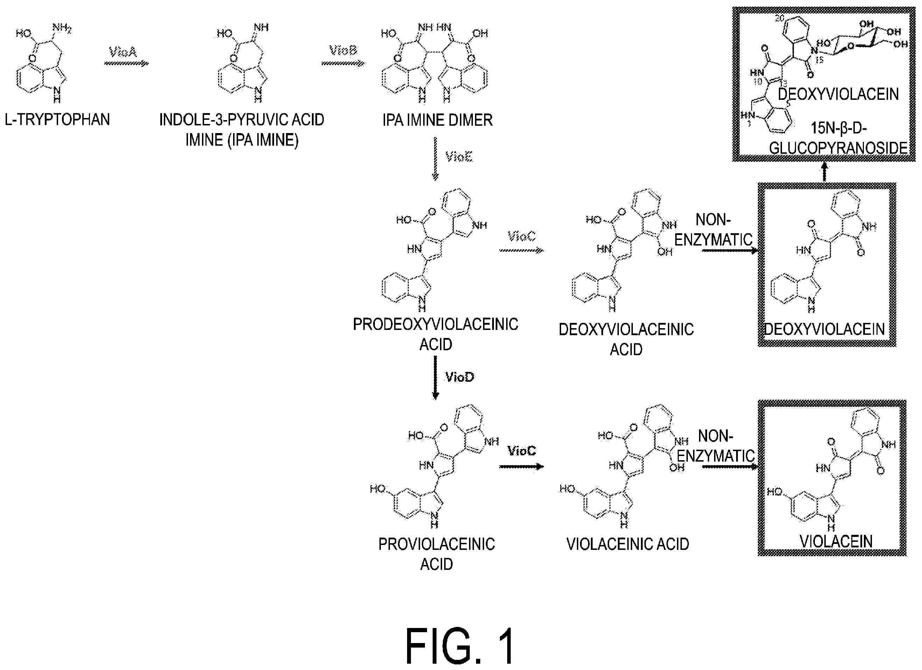

As illustrated in FIG. 1, VioA functions as an L-tryptophan oxidase and converts L-tryptophan into indole-3-pyruvic acid imine (IPA imine). VioB functions as an iminophenylpyruvate dimer synthase and converts IPA imine into an IPA imine dimer. VioE converts the IPA imine dimer into prodeoxyviolaceinic acid. VioD functions as a tryptophan hydroxylase and converts prodeoxyviolaceinic acid into proviolaceinic acid. VioC acts as a FAD-dependent monooxygenase and converts proviolaceinic acid into violaceinic acid. Then, violacein is non-enzymatically generated from violaceinic acid.

Deoxyviolacein, a structural analog of violacein, is non-enzymatically generated from deoxyviolaceinic acid through 4 enzymatic reactions except for VioD.

The present invention is based on the surprising finding that codons of 4 Vio genes required for biosynthesis of deoxyviolacein derived from Chromobacterium violaceum, that is, a VioA gene, a VioB gene, a VioC gene, and a VioE gene are optimized based on rose genomic information and introduced into cells of a plant containing L-tryptophan, thereby making it possible to create a transgenic plant with alteration of flower color, preferably alteration to a blue flower color.

The VioA gene used in the present invention is selected from the group consisting of the following polynucleotides:
(1-a) a polynucleotide consisting of a nucleotide sequence of SEQ ID NO: 1;
(1-b) a polynucleotide that hybridizes under stringent conditions with another polynucleotide consisting of a nucleotide sequence complementary to the nucleotide sequence of SEQ ID NO: 1, wherein the polynucleotide encodes a protein having a function of converting L-tryptophan into indole-3-pyruvic acid imine (IPA imine);
(1-c) a polynucleotide that encodes a protein consisting of an amino acid sequence of SEQ ID NO: 2;
(1-d) a polynucleotide that encodes a protein consisting of an amino acid sequence in which one or several amino acids are deleted, substituted, inserted, and/or added in the amino acid sequence of SEQ ID NO: 2, the protein having a function of converting L-tryptophan into indole-3-pyruvic acid imine (IPA imine); and
(1-e) a polynucleotide that encodes a protein having an amino acid sequence with 90% or more identity to the amino acid sequence of SEQ ID NO: 2, the protein having a function of converting L-tryptophan into indole-3-pyruvic acid imine (IPA imine).

The VioB gene used in the present invention is selected from the group consisting of the following polynucleotides:
(2-a) a polynucleotide consisting of a nucleotide sequence of SEQ ID NO: 3;
(2-b) a polynucleotide that hybridizes under stringent conditions with another polynucleotide consisting of a nucleotide sequence complementary to the nucleotide sequence of SEQ ID NO: 3, wherein the polynucleotide encodes a protein having a function of converting IPA imine into an IPA imine dimer;
(2-c) a polynucleotide that encodes a protein consisting of an amino acid sequence of SEQ ID NO: 4;
(2-d) a polynucleotide that encodes a protein consisting of an amino acid sequence in which one or several amino acids are deleted, substituted, inserted, and/or added in the amino acid sequence of SEQ ID NO: 4, the protein having a function of converting IPA imine into an IPA imine dimer; and
(2-e) a polynucleotide that encodes a protein having an amino acid sequence with 90% or more identity to the amino acid sequence of SEQ ID NO: 4, the protein having a function of converting IPA imine into an IPA imine dimer.

The VioC gene used in the present invention is selected from the group consisting of the following polynucleotides:
(3-a) a polynucleotide consisting of a nucleotide sequence of SEQ ID NO: 5;
(3-b) a polynucleotide that hybridizes under stringent conditions with another polynucleotide consisting of a nucleotide sequence complementary to the nucleotide sequence of SEQ ID NO: 5, wherein the polynucleotide encodes a protein having a function of converting prodeoxyviolaceinic acid into deoxyviolaceinic acid;
(3-c) a polynucleotide that encodes a protein consisting of an amino acid sequence of SEQ ID NO: 6;
(3-d) a polynucleotide that encodes a protein consisting of an amino acid sequence in which one or several amino acids are deleted, substituted, inserted, and/or added in the amino acid sequence of SEQ ID NO: 6, the protein having a function of converting prodeoxyviolaceinic acid into deoxyviolaceinic acid; and
(3-e) a polynucleotide that encodes a protein having an amino acid sequence with 90% or more identity to the amino acid sequence of SEQ ID NO: 6, the protein having a function of converting prodeoxyviolaceinic acid into deoxyviolaceinic acid.

The VioE gene used in the present invention is selected from the group consisting of the following polynucleotides:
(4-a) a polynucleotide consisting of a nucleotide sequence of SEQ ID NO: 7;
(4-b) a polynucleotide that hybridizes under stringent conditions with another polynucleotide consisting of a nucleotide sequence complementary to the nucleotide sequence of SEQ ID NO: 7, wherein the polynucleotide encodes a protein having a function of converting an IPA imine dimer into prodeoxyviolaceinic acid;
(4-c) a polynucleotide that encodes a protein consisting of an amino acid sequence of SEQ ID NO: 8;
(4-d) a polynucleotide that encodes a protein consisting of an amino acid sequence in which one or several amino acids are deleted, substituted, inserted, and/or added in the amino acid sequence of SEQ ID NO: 8, the protein having a function of converting an IPA imine dimer into prodeoxyviolaceinic acid; and
(4-e) a polynucleotide that encodes a protein having an amino acid sequence with 90% or more identity to the amino acid sequence of SEQ ID NO: 8, the protein having a function of converting an IPA imine dimer into prodeoxyviolaceinic acid.

An 5'-untranslated region (5'-UTR) derived from Arabidopsis thaliana-derived alcohol dehydrogenase (ADH) gene (SEQ ID NO: 12) can be added as a translation enhancer to the VioA gene, the VioB gene, the VioC gene, and the VioE gene, or a homolog thereof.

As used herein, the term "polynucleotide" refers to DNA or RNA.

As used herein, the term "stringent conditions" refers to conditions that allow specific binding between a polynucleotide or oligonucleotide and genomic DNA in a selective and detectable manner. Stringent conditions are defined by an appropriate combination of salt concentration, organic solvent (for example, formamide), temperature, and other known conditions. That is, stringency is increased by reducing the salt concentration, increasing the organic solvent concentration, or increasing the hybridization temperature. In addition, a post-hybridization washing condition affects stringency. This washing condition is also defined by salt concentration and temperature, and the stringency of the washing is increased by decreasing the salt concentration and increasing the temperature. Therefore, the term "stringent conditions" means conditions such that specific hybridization takes place only between nucleotide sequences with high identity, such as a degree of "identity" between the nucleotide sequences of about 80% or more, preferably about 90% or more, more preferably about 95% or more, further preferably 97% or more, and most preferably 98% or more, on average. Examples of the "stringent conditions" include conditions at a temperature of 60°C to 68°C, a sodium concentration of 150 to 900 mM, preferably 600 to 900 mM, and a pH of 6 to 8, and specific examples thereof include hybridization under conditions of 5 × SSC (750 mM NaCl, 75 mM trisodium citrate), 1% SDS, 5 × Denhardt solution 50% formaldehyde, and 42°C, and washing under conditions of 0.1 × SSC (15 mM NaCl, 1.5 mM trisodium citrate), 0.1% SDS, and 55°C.

The hybridization may be carried out according to a method known in the art or a similar method, such as the method described in Current Protocols in Molecular Biology (edited by Frederick M. Ausubel et al., 1987). When a commercially available library is to be used, the hybridization may be carried out according to the method described in the accompanying directions for use. The gene selected by hybridization may be naturally derived, such as plant-derived or non-plant-derived. The gene selected by the hybridization may be cDNA, genomic DNA, or chemically synthesized DNA.

In the present specification, the phrase "amino acid sequence in which one or several amino acids are deleted, substituted, inserted, and/or added" means an amino acid sequence in which any number of amino acids, for example, 1 to 20, preferably 1 to 5, and more preferably 1 to 3 amino acids are deleted, substituted, inserted, and/or added. Site-specific mutagenesis, a useful genetic engineering method as it allows introduction of specific mutations into specified sites, can be carried out according to the method described in Molecular Cloning: A Laboratory Manual, 2nd Ed., Cold Spring Harbor Laboratory Press, Cold Spring Harbor, N.Y., 1989, and the like. By expressing the mutant DNA using a suitable expression system, it is possible to obtain a protein consisting of an amino acid sequence in which one or several amino acids are deleted, substituted, inserted, and/or added.

The polynucleotide can be obtained by a method known to those skilled in the art, for example, a method of chemical synthesis using the phosphoramidite method, or a nucleic acid amplification method using a plant nucleic acid specimen as template and primers designed based on the nucleotide sequence of the target gene.

In the present specification, the term "identity" means the quantity (number) of amino acid residues or nucleotides constituting two chains in a polypeptide sequence (or amino acid sequence) or a polynucleotide sequence (or nucleotide sequence) that can be determined to be the same in the compatibility relationship between the amino acid residues or nucleotides, meaning the degree of sequence correlation between two polypeptide sequences or two polynucleotide sequences, and the term "identity" can be easily calculated. Numerous methods are known for measuring identity between two polynucleotide sequences or polypeptide sequences, and the term "identity" is well known to those skilled in the art (for example, see Lesk, A. M. (Ed.), Computational Molecular Biology, Oxford University Press, New York,

(1988); Smith, D. W. (Ed.), Biocomputing: Informatics and Genome Projects, Academic Press, New York, (1993); Grifin, A. M. & Grifin, H. G. (Ed.), Computer Analysis of Sequence Data: Part I, Human Press, New Jersey, (1994); von Heinje, G., Sequence Analysis in Molecular Biology, Academic Press, New York, (1987); and Gribskov, M. & Devereux, J. (Ed.), Sequence Analysis Primer, M-Stockton Press, New York, (1991)).

In addition, the numerical values for "identity" used in the present specification, unless otherwise specified, may be the numerical values calculated using an identity search program known to those skilled in the art but are preferably numerical values calculated using the ClustalW program of MacVector Application (version 18.2.5, Oxford Molecular Ltd., Oxford, England). In the present invention, the degree of "identity" between polynucleotide sequences or amino acid sequences is, for example, about 90% or more, preferably about 95% or more, more preferably about 97% or more, and most preferably about 98% or more.

The polynucleotide (nucleic acid, gene) of the present invention "encodes" a protein of interest. As used herein, the term "encodes" means that the protein of interest is expressed in a state of exhibiting its activity. In addition, the term "encodes" includes the meaning of both encoding a structural sequence (exon) that is a continuous section of the protein of interest, and encoding via an intervening sequence (intron).

A gene having a native nucleotide sequence can be obtained by, for example, analysis using a DNA sequencer. In addition, DNA encoding an enzyme having a modified amino acid sequence can be synthesized using common site-specific mutagenesis or PCR, based on DNA having the natural nucleotide sequence. For example, a DNA fragment to be modified may be obtained by restriction enzyme treatment of natural cDNA or genomic DNA, and used as template for site-specific mutagenesis or PCR using primers with the desired mutation, to obtain a DNA fragment having the desired modification. The DNA fragment having the mutation may then be linked with a DNA fragment encoding another portion of the target enzyme.

Alternatively, in order to obtain DNA encoding an enzyme consisting of a shortened amino acid sequence, DNA encoding an amino acid sequence longer than the target amino acid sequence, such as the full-length amino acid sequence, may be cut with a desired restriction enzyme, and if the obtained DNA fragment does not encode for the full target amino acid sequence, then a DNA fragment consisting of the sequence of the missing portion may be synthesized and linked thereto.

By expressing the obtained polynucleotide using a gene expression system in Escherichia coli or yeast and measuring the enzyme activity, it is possible to confirm that the obtained polynucleotide encodes a protein with the desired activity.

The vector of the present invention includes a VioA gene, a VioB gene, a VioC gene, a VioE gene, or any combination thereof, and the vector is typically an expression vector.

The expression vector of the present invention includes an expression control region (for example, a promoter, a terminator, and/or a replication origin) depending on the kind of the host to which the expression vector is to be introduced. Examples of the promoter that constitutively expresses a polynucleotide in a plant cell include a 35S promoter of cauliflower mosaic virus, an El₂35S promoter having two 35S promoter enhancer regions linked together, an rd29A gene promoter, an rbcS promoter, and a mac-1 promoter. For tissue-specific gene expression, a promoter for a gene expressed specifically in that tissue may be used.

The vector of the present invention preferably further includes a VioA gene and a promoter linked to the VioA gene and functions as a petal-specific promoter. Such a promoter is selected from, for example, a lisianthus-derived anthocyanidin synthase gene promoter(SEQ ID NO: 9), a morning glory-derived dihydroflavonol 4-reductase gene promoter (SEQ ID NO: 10), or a rose-derived chalcone synthase gene promoter (SEQ ID NO: 11).

The vector can be produced by a known method using a restriction enzyme, ligase, or the like. Transformation of a host plant with an expression vector can also be carried out according to a known method.

Under the current state of the art, a technique of introducing a vector according to the present invention into a plant and constitutively or tissue-specific expressing a polynucleotide in the vector can be used. The DNA can be introduced into a plant by a method known to those skilled in the art, such as the Agrobacterium method, binary vector method, electroporation method, PEG method or particle gun method.

In the present invention, the plant that can be used as a host is not particularly limited as long as the cell contains L-tryptophan, a starting substrate in biosynthesis of violaceins, but plants belonging to genus Rosaceae Rosa, Caryophyllaceae Dianthus, Compositae Chrysanthemum, or Liliaceae Lilium can be used, typically rose, carnation, chrysanthemum, or lily, and particularly preferably cultivated rose (scientific name: Rosa hybrida). The term "rose plant", as used herein, is a rose cultivar of Rosaceae Rosa (scientific name: Rosa hybrida), in terms of taxonomical classification. Roses are largely classified as Hybrid Tea, Floribunda and Polyantha roses based on their tree form and flower size. The type of rose plant used as a host for the present invention is not particularly limited, and any of the varieties or lines can be suitably used. Examples of rose varieties to be used as hosts include Ocean Song, Be Sweet, Nectarine, Lavande, Mondial, Arleene, WKS82, Noblesse, Rita Perfumera, Cool Water, Fame, Topless, and Peach Avalanche.

According to the present invention, a VioA gene, a VioB gene, a VioC gene, a VioE gene, or any combination thereof is introduced, and therefore a blue pigment, violaceins (for example, deoxyviolacein or a glycoside thereof) are accumulated in cells, thereby creating a transgenic plant with alteration of flower color, preferably a rose, carnation, chrysanthemum, or a lily plant. The altered flower color is preferably a blue flower color, for example, a flower color of the Blue group or the Violet-Blue group in the RHS color chart, and/or a flower color having a hue angle of 339.7° to 270.0°, preferably 315° or less in the CIEL^{*}a^{*}b^{*} color system.

The glycoside of deoxyviolacein can be represented, for example, by the following formula:

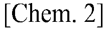

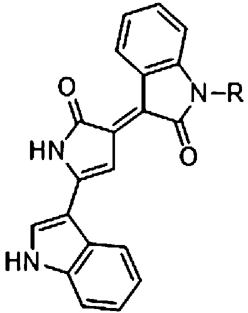

Here, in the formula, R may be, for example, a monosaccharide unit selected from an aldose such as glucose, mannose, galactose, fucose, rhamnose, arabinose, or xylose, ketose such as fructose, and glucuronic acid, a kind of or uronic acid. Alternatively, R may be a sugar chain formed by linking two or more monosaccharide units thereof.

Furthermore, the present invention also relates to the transgenic plant or its inbred or outbred progeny obtained above, a propagule, partial plant body, tissue, or cell thereof, or a cut flower or a processed form created from the cut flower (particularly, a cut flower processed product). Here, examples of the cut flower processed product include, but is not limited to, a pressed flower using the cut flower, a preserved flower, a dry flower, and a resin-sealed product.

Furthermore, the applicant has isolated and purified a blue pigment, an unknown compound accumulated in cells of a rose, in addition to deoxyviolacein, by introducing the VioA gene, the VioB gene, the VioC gene, and the VioE gene into the cells and succeeded in identifying the blue pigment as a compound (i.e., deoxyviolacein 15N-β-D-glucopyranoside) represented by the following formula.

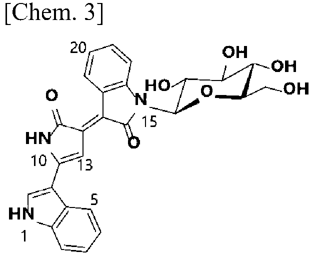

Therefore, in a further aspect, the present invention also relates to a method for producing deoxyviolacein or a glycoside thereof, particularly the novel compound (i.e., deoxyviolacein 15N-β-D-glucopyranoside), the method including the steps of: introducing the above-described genes according to the present invention or the vector according to the present invention into plant cells; and collecting the compound from the cells. The compounds can be collected from the plant cells using extraction techniques traditional in the art.

The present invention also relates to a transgenic plant or its inbred or outbred progeny, a propagule, partial plant body, tissue, or cell thereof, or a cut flower or a processed form created from the cut flower (particularly, a cut flower processed product), containing deoxyviolacein or a glycoside thereof (e.g., deoxyviolacein 15N-β-D-glucopyranoside).

Furthermore, the present invention relates to the novel compound (i.e., deoxyviolacein 15N-β-D-glucopyranoside) per se. The compound according to the present invention can be used as a dye for dyeing a textile product in a blue color. The compound of the present invention can be used as a colorant for coloring a medicament, cosmetic, food, or beverage product in a blue color.

Moreover, since violaceins are known to possess broad-spectrum antimicrobial activity against organisms such as Staphylococcus aureus, Bacillus spp., Streptococcus spp., Mycobacterium, Neisseria, and Pseudomonas, as well as antioxidant activity, antitumor activity, antiviral activity, and antiprotozoal activity, the compound (i.e., deoxyviolacein 15N-β-D-glucopyranoside) according to the present invention can be used as such an active ingredient in a medicine or cosmetic.

Hereinafter, the present invention will be described in detail with reference to Examples.

### Example 1

### [Example 1: Acquisition of Full-Length Deoxyviolacein Synthase Gene]

The four genes VioA, VioB, VioC, and VioE required for synthesis of deoxyviolacein were codon-optimized for expression in rose, based on the sequence of Chromobacterium violaceum (VioA: WP_011136821.1, VioB: AVR55196.1, VioC: WP_011136819, and VioE: WP_011136817.1) published in the National Center for Biotechnology Information of the National Library of Medicine, to obtain full-length sequences by artificial gene synthesis, which were named RhcVioA, RhcVioB, RhcVioC, and RhcVioE, respectively. The sequences are as follows.

The nucleotide sequence of RhcVioA is as follows.

atgaagcacagttcggatatctgcatagttggggccggtatatcaggacttacatgtgcctcgcatctcttggacagtccagcatgtaggggt
ctttctctccgcatctttgacatgcaacaagaagccggaggccgaattaggtccaagatgcttgacggaaaagcctcgattgagttgggtgct
ggtaggtatagccctcagttgcatcctcacttccaatctgccatgcagcactacagtcagaagagtgaggtctacccgtttacgcaactcaag
ttcaagagccacgttcaacagaagctcaaacgcgctatgaacgagttgagtcccagattgaaggagcatgggaaagagtccttcttgcagtt
cgtgtcccgataccaagggcatgatagcgcagtaggcatgattcgctcaatgggctatgatgccctttttcttcccgacatttccgccgagatg
gcatatgacatagtgggcaaacaccccgaaatccagtctgttaccgacaatgacgcaaaccagtggtttgcagcagagactggctttgcag
gactgattcaggggattaaggcaaaggttaaggctgctggagcccgtttctcgctgggttatcggcttctttcagttcgcacagatggggatg

gctacttgttacaattagcaggtgatgatggttggaaactcgagcacagaactaggcacttgatactcgccattccaccgtctgctatggctgg
cctgaatgtcgactttcctgaggcttggtctggggctagatacgggagcttgcctctcttcaagggatttctgacctacggtgaaccatggtgg
ctcgactacaaacttgacgatcaagtcttgatcgtggacaatccgcttaggaagatctacttcaagggggataagtacctcttcttctacaccg
actccgaaatggccaactactggagaggttgcgtagctgagggtgaggacgggtacttggaacagatccgtacacatctcgcttctgcgct
agggatcgtgcgtgagcgtattcctcaaccgttagctcacgtgcacaaatactgggcacatggtgtcgagttttgtcgggattctgacattgac
cacccttctgccctctcacatcgggattcaggaattatcgcctgtagcgatgcatacactgagcattgtggttggatggaaggagggttacta
agtgctcgggaagcttcacgtttgctactgcaacgaattgcagcctaa (SEQ ID NO: 1)

The amino acid sequence of RhcVioA is as follows.

MKHSSDICIVGAGISGLTCASHLLDSPACRGLSLRIFDMQQEAGGRIRSKMLDGKASIEL
GAGRYSPQLHPHFQSAMQHYSQKSEVYPFTQLKFKSHVQQKLKRAMNELSPRLKEHGK
ESFLQFVSRYQGHDSAVGMIRSMGYDALFLPDISAEMAYDIVGKHPEIQSVTDNDANQ
WFAAETGFAGLIQGIKAKVKAAGARFSLGYRLLSVRTDGDGYLLQLAGDDGWKLEHR
TRHLILAIPPSAMAGLNVDFPEAWSGARYGSLPLFKGFLTYGEPWWLDYKLDDQVLIVD
NPLRKIYFKGDKYLFFYTDSEMANYWRGCVAEGEDGYLEQIRTHLASALGIVRERIPQP
LAHVHKYWAHGVEFCRDSDIDHPSALSHRDSGIIACSDAYTEHCGWMEGGLLSAREAS
RLLLQRIAA* (SEQ ID NO: 2)

The nucleotide sequence of RhcVioB is as follows.

atgtcgatcttggactttcctcgtatccacttcaggggttgggctcgggtcaatgctccgactgccaatcgcgatccgcatggacacattgata
tggcgtctaatacagttgcaatggcaggggagccgtttgatttggccagacatccaactgagtttcacagacatttgcgttctcttggtccccg
ctttggcctcgatggccgagccgatcccgaaggaccgtttagtctggcagaaggctacaacgctgccggtaacaaccacttctcttgggag
agtgccacggtgtctcacgttcagtgggacgggggtgaggcagaccgcggagatggactggtcggtgctagactcgctctttggggtcatt
acaatgattaccttcgcacaacgtttaatcgggctaggtgggtggatagcgatccaaccagaagagacgctgcacagatctatgctggcca
attcaccatctcgcctgcaggtgctggacctggaacgccgtggctcttcacagctgacatcgacgacagtcatggtgctcgatggaccaga
ggtgggcatattgccgagcgtggtggacatttcttggacgaggagtttggcttggctcgcttgtttcaattctccgtgccaaaggaccatcctc
acttcttgtttcaccctggtccctttgactcagaagcctggcggaggctccagcttgctctcgaggatgacgacgttctcgggttgactgttca
gtacgcattgttcaacatgtctactcctccgcaacccaattccccggttttccatgatatggtgggggtagtcggactttggcgtaggggcgaa
cttgccagttatcctgcagggcggttacttcggcctaggcaacccggtttgggtgacttgaccttgcgtgtgaatggcggtagagtcgctctg
aaccttgcctgtgcaattccattctctactcgagccgctcagccgtcggcaccggaccgcctcacaccggacttaggggcaaagctgcctct
tggggacttacttctcagggacgaggatggtgccttgctggcaagggtcccacaggccctttaccaggactactggacgaatcatgggata
gttgacttgcctttgcttagagaaccacgcggctctctaaccttaagtagcgagctagccgaatggcgggagcaggattgggtcactcagtc
agatgccagtaacctttacctcgaggctcctgatcgaagacacggaagattttttcccgagtcgattgcccttcgctcatacttccgtggggag
gcaagagccagacctgacattccccaccgtatcgaaggcatgggcctcgttggtgtggaatcacgtcaagacggggatgccgcagagtg
gcggttgacagggttaaggcctggtcctgctcgtatcgtgctagatgatggggccgaggccatacccctgagggtactcccggacgattgg
gcattagacgatgccacagtcgaggaggtagactacgcttttttgtaccgtcatgttatggcgtattacgaactcgtgtacccgttcatgagcg
acaaagtgttcagcctagccgacaggtgcaagtgcgaaacttacgcccgccttatgtggcagatgtgtgatccgcaaaaccgcaacaagtc
ctactacatgccctctacccgagagttgtctgctccgaaagctcgactgttcctcaagtacctcgctcacgtggagggccaagctagactcca
agcacctccacccgcaggaccggctcggatagagagtaaggcacaactcgcagctgaacttagaaaggctgtggatttggaattaagcgt

gatgcttcagtacttgtatgccgcttactccattcccaactatgcacagggtcagcaacgtgtcagggacggggcttggaccgccgagcaac
tgcaacttgcttgtgggtctggtgatagacgacgtgacggaggtatcagggccgctttactagagatagcccacgaggagatgatccacta
cttggtcgttaacaacttgttgatggcccttggcgaacccttttacgccggagtaccgttgatgggcgaagcagcacgtcaagcattcggatt
ggatacagagttcgcgttagagcctttcagtgagtcaactctcgcacgtttcgtccgacttgaatggcctcacttcattcctgctccggggaag
tcaattgctgactgctacgccgctattcggcaagcctttctggatttaccggacctctttggtggagaagctggtaaacgcggcggagagca
ccacctatttctcaatgagctgacaaaccgtgcacatcctgggtatcagttggaggtgtttgacagggactccgctctcttcggtattgcctttgt
taccgatcaaggggaagggggtgccttagactcacctcactatgagcattcccatttccagaggctccgtgagatgtccgcacgcatcatgg
cccaatctgctccattcgaacctgccctacctgcactccgtaaccccgttttggatgagtcaccagggtgtcaaagggttgccgacggaaga
gccagggcactcatggctctgtaccaaggcgtttatgagcttatgttcgcaatgatggcccaacactttgcagtgaaaccactcggtagtcttc
gaaggtcacgtcttatgaatgcagccattgacctcatgactgggcttttgcggcctctcagctgtgcattgatgaatttgccttctgggattgca
ggacgaactgccggtccaccattgccgggcccagttgacacccgttcgtacgatgactacgccttggggtgtcgtatgctcgctcgccgct
gcgagcggctcctggaacaggcaagcatgttggagcctgggtggctgccagatgcacagatggagctgctggatttctaccgaaggcag
atgctcgatctagcttgtggaaagttatcccgggaggcctaa (SEQ ID NO: 3)

The amino acid sequence of RhcVioB is as follows.

MSILDFPRIHFRGWARVNAPTANRDPHGHIDMASNTVAMAGEPFDLARHPTEFHRHLRS
LGPRFGLDGRADPEGPFSLAEGYNAAGNNHFSWESATVSHVQWDGGEADRGDGLVGA
RLALWGHYNDYLRTTFNRARWVDSDPTRRDAAQIYAGQFTISPAGAGPGTPWLFTADI
DDSHGARWTRGGHIAERGGHFLDEEFGLARLFQFSVPKDHPHFLFHPGPFDSEAWRRLQ
LALEDDDVLGLTVQYALFNMSTPPQPNSPVFHDMVGVVGLWRRGELASYPAGRLLRPR
QPGLGDLTLRVNGGRVALNLACAIPFSTRAAQPSAPDRLTPDLGAKLPLGDLLLRDEDG
ALLARVPQALYQDYWTNHGIVDLPLLREPRGSLTLSSELAEWREQDWVTQSDASNLYL
EAPDRRHGRFFPESIALRSYFRGEARARPDIPHRIEGMGLVGVESRQDGDAAEWRLTGL
RPGPARIVLDDGAEAIPLRVLPDDWALDDATVEEVDYAFLYRHVMAYYELVYPFMSDK
VFSLADRCKCETYARLMWQMCDPQNRNKSYYMPSTRELSAPKARLFLKYLAHVEGQA
RLQAPPPAGPARIESKAQLAAELRKAVDLELSVMLQYLYAAYSIPNYAQGQQRVRDGA
WTAEQLQLACGSGDRRRDGGIRAALLEIAHEEMIHYLVVNNLLMALGEPFYAGVPLMG
EAARQAFGLDTEFALEPFSESTLARFVRLEWPHFIPAPGKSIADCYAAIRQAFLDLPDLFG
GEAGKRGGEHHLFLNELTNRAHPGYQLEVFDRDSALFGIAFVTDQGEGGALDSPHYEH
SHFQRLREMSARIMAQSAPFEPALPALRNPVLDESPGCQRVADGRARALMALYQGVYE
LMFAMMAQHFAVKPLGSLRRSRLMNAAIDLMTGLLRPLSCALMNLPSGIAGRTAGPPL
PGPVDTRSYDDYALGCRMLARRCERLLEQASMLEPGWLPDAQMELLDFYRRQMLDLA
CGKLSREA* (SEQ ID NO: 4)

The nucleotide sequence of RhcVioC is as follows.

atgaagcgtgcaatcattgtgggaggaggacttgcagggggcttgacagctatctatctagccaaacgcggttatgaggtccatgtggttga
gaaacgtggtgaccctctacgtgacttgagcagttacgtggatgtcgtttcttctagagctatcggtgtgtccatgacagttcgtgggatcaaat
cagtcctcgctgctgggattcctcgtgctgaactcgatgcatgtggtgaaccgatagtcgctatggcattcagtgtgggagggcagtatcgg
atgagggagttgaagcccttagaggactttcgcccactgagcttgaatcgagctgcctttcaaaagctgttgaacaagtacgccaacttagct

ggagtacgctactacttcgagcacaaatgcctcgatgtggatcttgatggaaagtccgttctcattcagggcaaagatgggcaacctcaaag
gctgcaaggtgacatgatcatcggtgccgatggtgcacactctgccgttcgccaggctatgcaatccggcttgagacgtttcgagttccaac
agaccttctttcgccatgggtacaagaccttggttcttccagacgctcaagcactaggttacaggaaggatacgctgtactttttcggcatggat
agtgggggactttttgccggtagagctgcaaccataccggatgggtccgtaagcattgccgtttgtctcccatactcgggctcaccttcactg
actactactgacgagcctactatgcgagcctttttcgaccgttactttggcggtttacctagagacgcacgagacgaaatgcttcgtcagttcct
tgccaaaccgtctaatgacctcattaacgttcggtcttcgactttccactacaagggtaacgtgctcctcttgggagatgcagcacatgccaca
gcaccctttttggggcaagggatgaatatggccttagaagacgcgagaacgttcgttgagctcttggaccgacatcaaggggatcaggaca
aagcctttcccgagtttaccgagttgaggaaggtccaagctgacgcaatgcaagacatggctagggccaactatgacgtgctttcatgttcg
aaccccattttcttcatgagggcacggtacacacggtacatgcactctaagtttccgggcttgtatccgcctgatatggccgagaagctctact
tcaccagtgaaccatacgatagacttcagcagatacagcggaagcagaatgtgtggtacaagattggtagggtcaattaa (SEQ ID
NO: 5)

The amino acid sequence of RhcVioC is as follows.

MKRAIIVGGGLAGGLTAIYLAKRGYEVHVVEKRGDPLRDLSSYVDVVSSRAIGVSMTV
RGIKSVLAAGIPRAELDACGEPIVAMAFSVGGQYRMRELKPLEDFRPLSLNRAAFQKLL
NKYANLAGVRYYFEHKCLDVDLDGKSVLIQGKDGQPQRLQGDMIIGADGAHSAVRQA
MQSGLRRFEFQQTFFRHGYKTLVLPDAQALGYRKDTLYFFGMDSGGLFAGRAATIPDG
SVSIAVCLPYSGSPSLTTTDEPTMRAFFDRYFGGLPRDARDEMLRQFLAKPSNDLINVRS
STFHYKGNVLLLGDAAHATAPFLGQGMNMALEDARTFVELLDRHQGDQDKAFPEFTE
LRKVQADAMQDMARANYDVLSCSNPIFFMRARYTRYMHSKFPGLYPPDMAEKLYFTS
EPYDRLQQIQRKQNVWYKIGRVN* (SEQ ID NO: 6)

The nucleotide sequence of RhcVioE is as follows.

atggagaatcgcgaaccaccgttgttgccggctagatggtccagtgcatacgtgtcgtactggtcacctatgctacctgacgaccaacttacc
tctgggtactgttggttcgactacgaacgggacatctgcaggatagacggcctgtttaacccgtggagtgagcgcgatacaggctatcgcct
ctggatgtccgaggtagggaatgctgcatctggaaggacctggaagcagaaggttgcctatgggagagaacggactgcattgggtgagc
agctttgtgagcgtccactggacgatgagacaggtccctttgccgagcttttccttcctcgagacgtcttgcgaagactcggtgctcgtcacat
tgggcgtagggttgtcttaggtcgtgaagccgatggatggcgctaccaacgtcctggcaaaggtcctagcactctctacctcgatgcagcct
ctgggactccgttacggatggttacgggagatgaggctagcagggcatcgttgcgagacttcccaaacgtgtcagaagccgagattcccg
atgctgtgtttgctgccaagagataa (SEQ ID NO: 7)

The amino acid sequence of RhcVioE is as follows.

MENREPPLLPARWSSAYVSYWSPMLPDDQLTSGYCWFDYERDICRIDGLFNPWSERDT
GYRLWMSEVGNAASGRTWKQKVAYGRERTALGEQLCERPLDDETGPFAELFLPRDVL
RRLGARHIGRRVVLGREADGWRYQRPGKGPSTLYLDAASGTPLRMVTGDEASRASLRD
FPNVSEAEIPDAVFAAKR* (SEQ ID NO: 8)

### [Example 2: Transient Expression of Deoxyviolacein Synthetic Gene Cluster in Rose Petal]

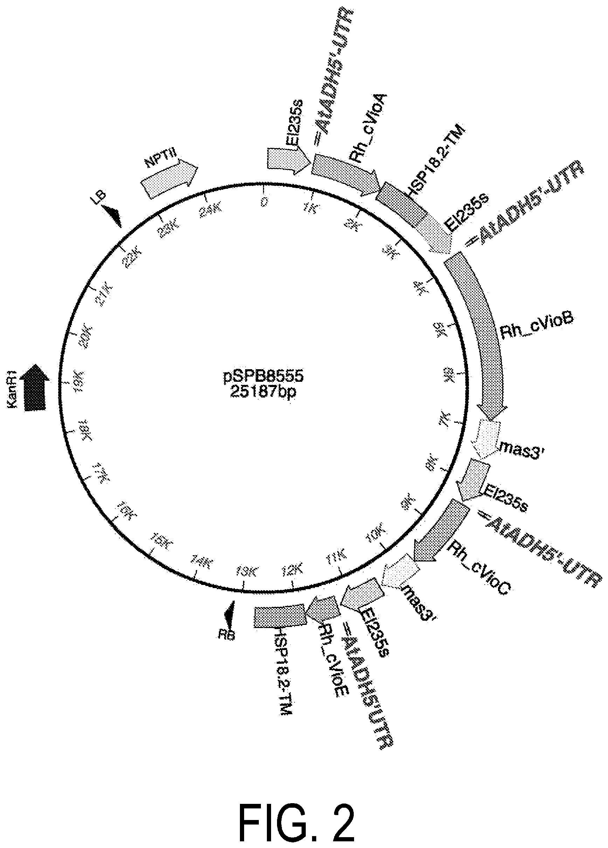

In order to confirm whether the RhcVioA, RhcVioB, RhcVioC, and RhcVioE genes in the deoxyviolacein synthetic gene cluster have an activity of synthesizing deoxyviolacein in rose petals, a binary vector pSPB8555 (FIG. 2) with the four genes introduced was constructed. This vector has pBINPLUS as a basic backbone and contains the following four expression cassettes.
(1) El₂35S promoter, C. violaceum-derived codon usage-modified RhcVioA full-length cDNA (SEQ ID NO: 1) (with addition of 5'-UTR derived from Arabidopsis thaliana-derived ADH gene (SEQ ID NO: 12), and Arabidopsis thaliana-derived HSP terminator
(2) El₂35S promoter, C. violaceum-derived codon usage-modified RhcVioB full-length cDNA (SEQ ID NO: 3) (with addition of 5'-UTR derived from Arabidopsis thaliana-derived ADH gene (SEQ ID NO: 12), and Agrobacterium-derived mannopine synthase gene (MAS) terminator
(3) El₂35S promoter, C. violaceum-derived codon usage-modified RhcVioC full-length cDNA (SEQ ID NO: 5), and Agrobacterium-derived MAS terminator
(4) El₂35S promoter, C. violaceum-derived codon usage-modified RhcVioE full-length cDNA (SEQ ID NO: 7) (with addition of 5'-UTR derived from Arabidopsis thaliana-derived ADH gene (SEQ ID NO: 12)), and Arabidopsis thaliana-derived HSP terminator

This binary vector constitutively expresses VioA, VioB, VioC, and VioE of C. violaceum in plants.

The pSPB8555 thus prepared was introduced into Agrobacterium tumefaciens, and the resultant was transiently introduced into rose petals (varieties: Ocean Song (OS), Be Sweet (BS), Nectarine (NT), Lavande (LA), Mondial (MD), Arleene (AL), and WKS82) according to a known method of vacuum infiltration described in, for example, Non-Patent Document 1. It was confirmed that the flower color changed to blue by storing in a dark place at 24°C for about 7 days. The blue-changed portion of the sample other than WKS82/8555 was cut out, about 0.1 g of the sample was freeze-dried, 50% acetonitrile containing 1 mL of 0.1% TFA was then added, and the components were extracted by sonication for 20 minutes and centrifuged at 10°C for 3,600 rotations for 10 minutes. The obtained supernatant was filtered through a filter, and then a reaction product was detected by HPLC (prominence LC-20AD manufactured by Shimadzu Corporation) under the following conditions.
Column used: Shim-pack FC-ODS 4.6 × 150 mm
Solvent solution A: 0.1% TFA
Solvent solution B: 90% acetonitrile containing 0.1% TFA
Mobile phase conditions: a linear gradient from A:B = 80:20 at 0 min to A:B = 30:70 at 10 min, followed by retention for 6 min; then a linear gradient back to A:B = 80:20 by 17 min, followed by retention for 11 min until 28 min
Flow rate: 0.6 mL/min
Column temperature: 40°C

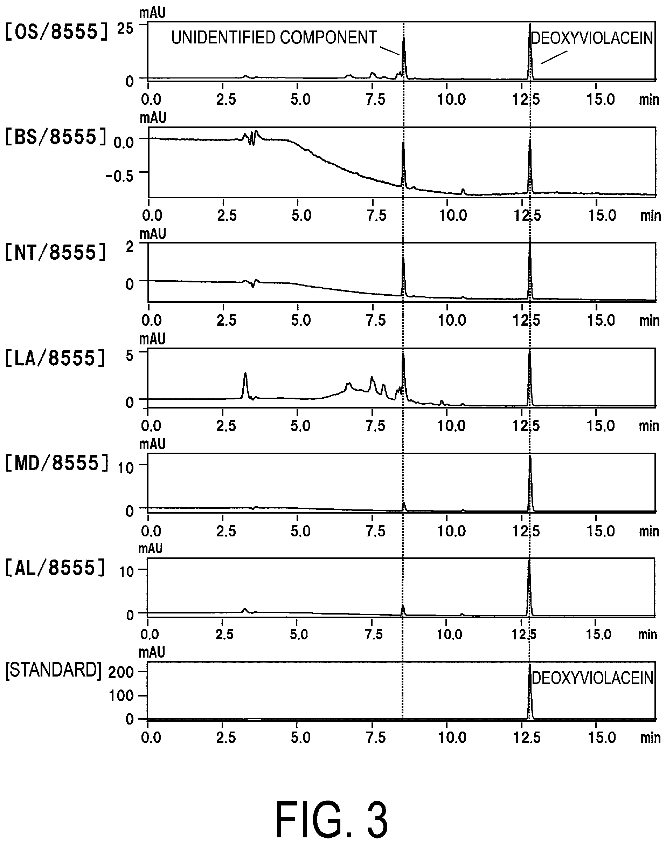

As an absorption spectrum, an absorbance at 570 nm was measured using a photodiode array (SPD-M20A). Under the conditions, deoxyviolacein was eluted at about 12.8 minutes. As illustrated in FIG. 3, an unidentified component (retention time: 8.5 minutes) was detected in addition to deoxyviolacein expected in this sample.

### [Example 3: Transient Expression of Deoxyviolacein Synthetic Gene Cluster in Carnation Petal]

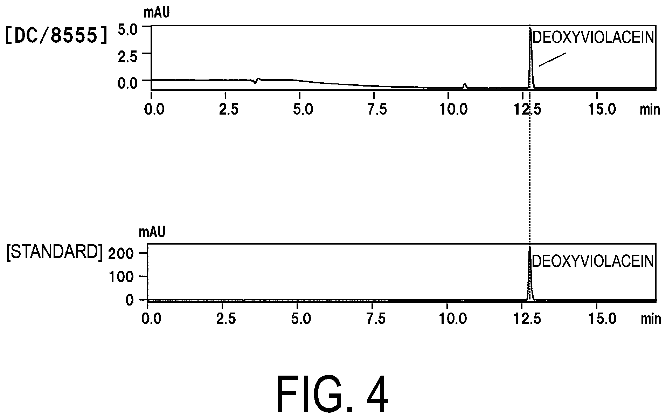

In order to confirm whether the RhcVioA, RhcVioB, RhcVioC, and RhcVioE genes in the deoxyviolacein synthetic gene cluster have an activity to synthesize deoxyviolacein in a carnation petal, a binary vector pSPB8555 (FIG. 2) was introduced into Agrobacterium tumefaciens in the same manner as in Example 2, and the resultant was transiently introduced into the carnation petal (variety: Durcal) by vacuum infiltration. As a result, the expected deoxyviolacein was detected in the carnation as seen in FIG. 4.

### [Example 4: Expression of Deoxyviolacein Synthetic Gene Cluster in Rose]

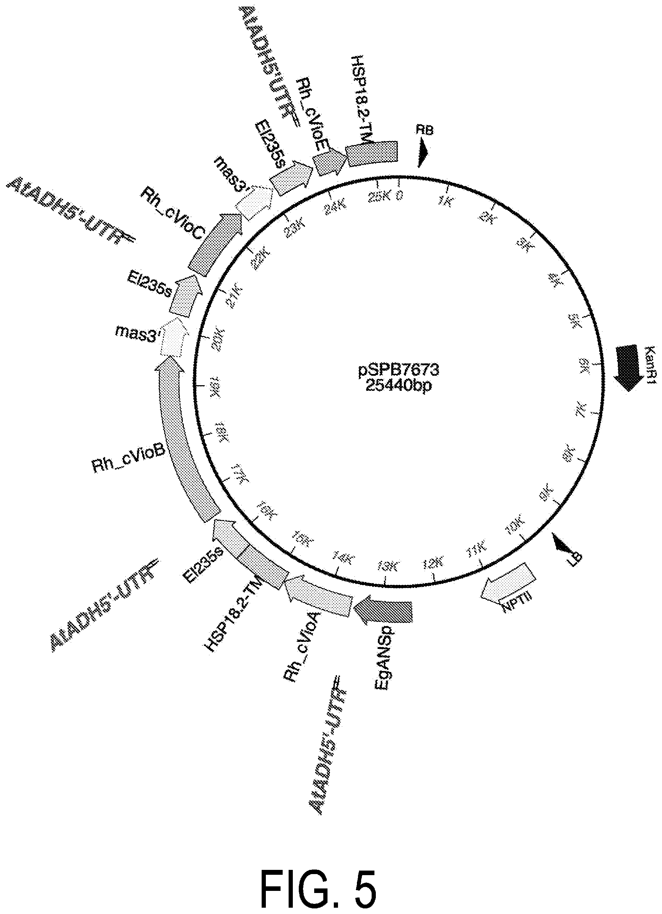

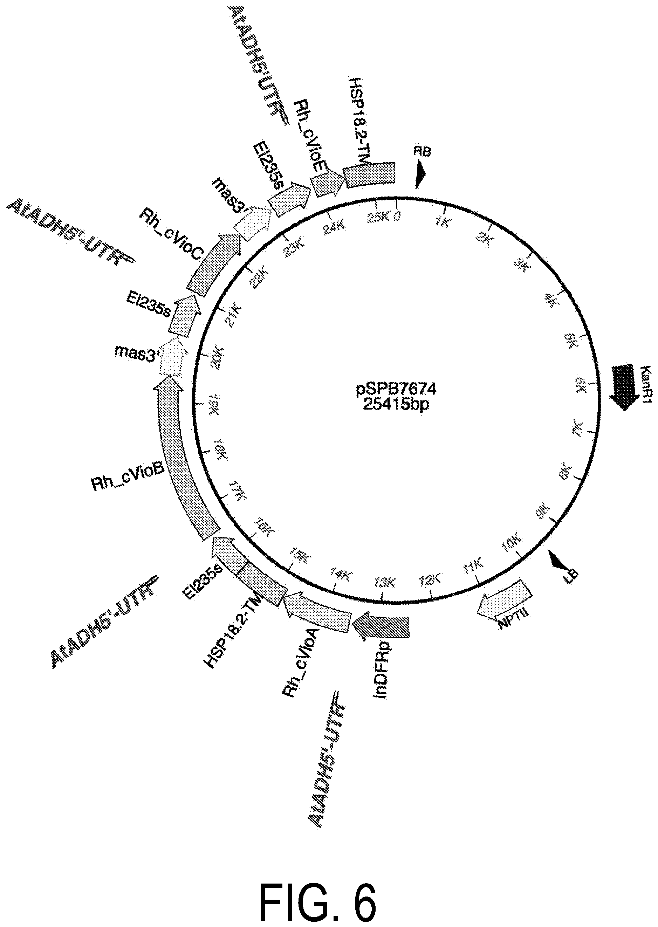

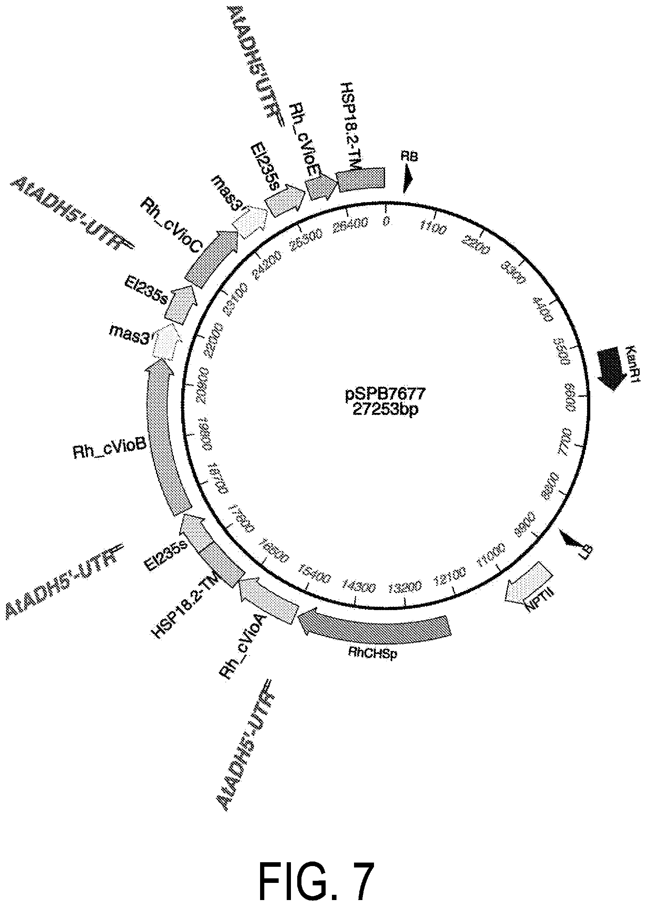

In order to confirm whether the RhcVioA, RhcVioB, RhcVioC, and RhcVioE genes in the deoxyviolacein synthetic gene cluster have an activity of synthesizing deoxyviolacein in roses, binary vectors pSPB8555 (FIG. 2), pSPB7673 (FIG. 5), pSPB7674 (FIG. 6), and pSPB7677 (FIG. 7) with the four genes introduced were constructed. The vectors each have pBINPLUS as a basic backbone and four expression cassettes in the following combinations.

### [pSPB8555]

As described in Example 2.

### [pSPB7673]

(1) Lisianthus-derived anthocyanidin synthase gene promoter (SEQ ID NO: 9), C. violaceum-derived codon usage-modified RhcVioA full-length cDNA (SEQ ID NO: 1) (with addition of 5'-UTR derived from Arabidopsis thaliana-derived ADH gene (SEQ ID NO: 12), and Arabidopsis thaliana-derived HSP terminator
(2) El₂35S promoter, C. violaceum-derived codon usage-modified RhcVioB full-length cDNA (SEQ ID NO: 3) (with addition of 5'-UTR derived from Arabidopsis thaliana-derived ADH gene (SEQ ID NO: 12), and Agrobacterium-derived mannopine synthase gene (MAS) terminator
(3) El₂35S promoter, C. violaceum-derived codon usage-modified RhcVioC full-length cDNA (SEQ ID NO: 5), and Agrobacterium-derived MAS terminator
(4) El₂35S promoter, C. violaceum-derived codon usage-modified RhcVioE full-length cDNA (SEQ ID NO: 7) (with addition of 5'-UTR derived from Arabidopsis thaliana-derived ADH gene (SEQ ID NO: 12), and Arabidopsis thaliana-derived HSP terminator

### [pSPB7674]

(1) Morning glory-derived dihydroflavonol 4-reductase gene promoter (SEQ ID NO: 10), C. violaceum-derived codon usage-modified RhcVioA full-length cDNA (SEQ ID NO: 1) (with addition of 5'-UTR derived from Arabidopsis thaliana-derived ADH gene (SEQ ID NO: 12), and Arabidopsis thaliana-derived HSP terminator
(2) El₂35S promoter, C. violaceum-derived codon usage-modified RhcVioB full-length cDNA (SEQ ID NO: 3) (with addition of 5'-UTR derived from Arabidopsis thaliana-derived ADH gene (SEQ ID NO: 12), and Agrobacterium-derived mannopine synthase gene (MAS) terminator
(3) El₂35S promoter, C. violaceum-derived codon usage-modified RhcVioC full-length cDNA (SEQ ID NO: 5), and Agrobacterium-derived MAS terminator
(4) El₂35S promoter, C. violaceum-derived codon usage-modified RhcVioE full-length cDNA (SEQ ID NO: 7) (with addition of 5'-UTR derived from Arabidopsis thaliana-derived ADH gene (SEQ ID NO: 12), and Arabidopsis thaliana-derived HSP terminator

### [pSPB7677]

(1) Rose-derived chalcone synthase gene promoter (SEQ ID NO: 11), C. violaceum-derived codon usage-modified RhcVioA full-length cDNA (SEQ ID NO: 1) (with addition of 5'-UTR derived from Arabidopsis thaliana-derived ADH gene (SEQ ID NO: 12), and Arabidopsis thaliana-derived HSP terminator
(2) El₂35S promoter, C. violaceum-derived codon usage-modified RhcVioB full-length cDNA (SEQ ID NO: 3) (with addition of 5'-UTR derived from Arabidopsis thaliana-derived ADH gene (SEQ ID NO: 12), and Agrobacterium-derived mannopine synthase gene (MAS) terminator
(3) El₂35S promoter, C. violaceum-derived codon usage-modified RhcVioC full-length cDNA (SEQ ID NO: 5), and Agrobacterium-derived MAS terminator
(4) El₂35S promoter, C. violaceum-derived codon usage-modified RhcVioE full-length cDNA (SEQ ID NO: 7) (with addition of 5'-UTR derived from Arabidopsis thaliana-derived ADH gene (SEQ ID NO: 12), and Arabidopsis thaliana-derived HSP terminator

This binary vector constitutively expresses a VioA gene, a VioB gene, a VioC gene, and a VioE gene of C. violaceum in plants.

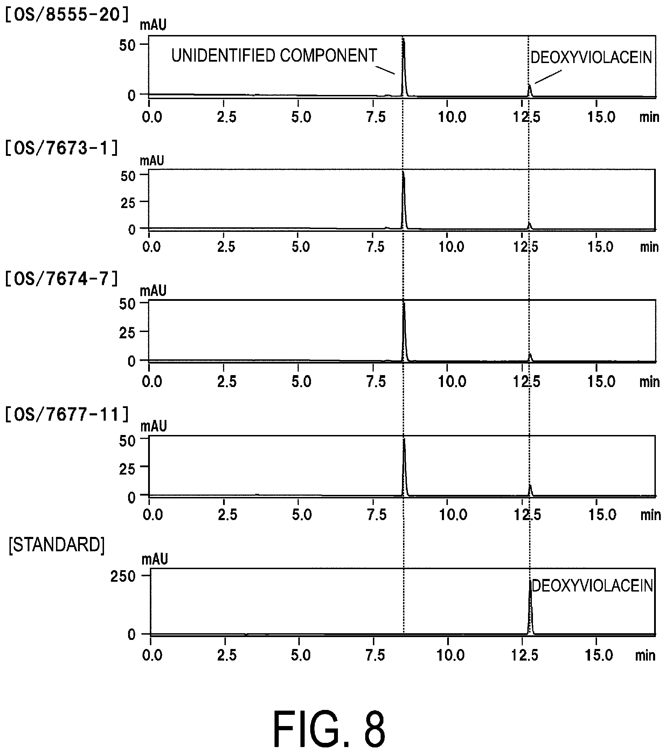

The pSPB8555, pSPB7673, pSPB7674, and pSPB7677 thus produced were introduced into a blue categoly variety "Ocean Song". About 0.2 g of the resulting rose transgenic callus was freeze-dried, 50% acetonitrile containing 1 mL of 0.1% TFA was then added, and the components were extracted by sonication for 20 minutes and centrifuged at 5°C for 15,000 rotations for 5 minutes. The obtained supernatant was filtered through a filter, and then a reaction product was detected by HPLC (prominence LC-20AD manufactured by Shimadzu Corporation) under the conditions described in Example 2. As a result, as seen in FIG. 8, an unidentified component (retention time: 8.5 minutes) was detected as a main component in the rose transgenic callus.

### [Example 5: Isolation of Unidentified Component]

### 1. Preparation of Extract from Petals

In the same manner as in Example 2, a binary vector pSPB8555 (FIG. 2) was introduced into Agrobacterium tumefaciens, and the bacteria were transiently introduced into rose petals (cultivar: Ocean Song) by vacuum infiltration. Portions exhibiting a blue color change were excised and stored frozen at -30°C.

Frozen petals (62.6 g, wet weight) were pulverized into a powder, and 300 mL of an 80% aqueous methanol solution was added thereto, followed by ultrasonic irradiation at room temperature for 40 minutes. After separating the extract from the residue, 300 mL of an 80% aqueous methanol solution was added to the residue, and the above extraction procedure was repeated.

Furthermore, 200 mL of 80% methanol was added to the residue, and the extraction operation was repeated to obtain a total of 800 mL of extract. The combined extract was concentrated to 150 mL using a rotary evaporator while heating at a temperature not exceeding 40°C.

### 2. Crude Purification of Extract Using Sep-Pak

A Sep-Pak C18 Plus Long cartridge (manufactured by Waters Corporation) was equilibrated with 10 mL of methanol followed by 10 mL of water, after which 10 mL of the crude pigment extract was loaded thereon. The pigments were then eluted sequentially with 10 mL each of water, 40% methanol (MeOH), 60% methanol, and 80% methanol.

As a result, a blue pigment (Compound (I)) was eluted with 60% methanol, and a blue pigment (Compound (II)) was eluted with 80% methanol. The purification using the Sep-Pak cartridge was carried out in a total of 17 runs.

### 3. Preparative HPLC Conditions for Blue Pigment Fractions Obtained by Sep-Pak

In the preparative high-performance liquid chromatography (HPLC) of the blue pigment fractions obtained by Sep-Pak purification, the following conditions were commonly employed.

The HPLC system comprised a degasser (DGU-20A), an autosampler (SIL-10AP), two pumps (LC-6AD ×2), a fraction collector (FRC-10A), a system controller (CBM-20A), a photodiode array detector (SPD-M20A), a column oven (CTO-20AC), and data processing software (LabSolutions, version 5.97), all manufactured by Shimadzu Corporation.

The column used was an Inertsil ODS-4 column (manufactured by GL Sciences Inc.) having dimensions of 20 mm inner diameter × 250 mm length. The mobile phase consisted of a binary gradient system of (A) a 0.1% aqueous formic acid solution and (B) acetonitrile. The flow rate was set to 8.0 mL/min, and the column oven temperature was maintained at 40°C.

The gradient elution profile was appropriately modified depending on the sample.

### 4. Preparative HPLC of Compound (I)

The 60% methanol (MeOH) fraction obtained by Sep-Pak purification was concentrated using a SpeedVac concentrator (manufactured by Thermo Scientific), whereby a precipitate was formed. Methanol was added until the precipitate was completely dissolved.

The resulting solution was subjected to preparative HPLC in portions of 1.5 mL each. The gradient conditions were as follows: solvent B, 20% to 30% (0 to 0.05 min), 30% hold (0.05 to 25 min), 30% to 100% (25 to 26 min), 100% to 100% (26 to 27 min), 100% to 20% (27 to 28 min), and 20% hold (28 to 38 min).

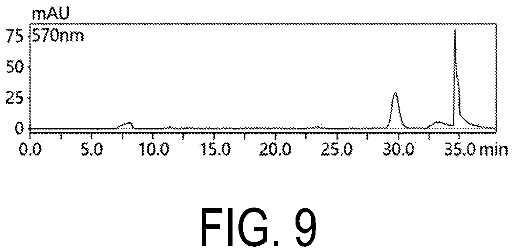

Absorbance at 570 nm was used as a purification indicator. Fractions having retention times of 29.2 to 30.3 minutes in the chromatogram shown in FIG. 9 were collected, and the organic solvent was removed using a SpeedVac concentrator, followed by lyophilization.

Compound (I) was obtained as a blue amorphous solid in an amount of 1.5 mg, corresponding to a yield of 2.4 × 10⁻³%.

The structure of this compound was determined by NMR, LC-MS, and the like to be represented by the following formula (I) (C₂₆H₂₃N₃O₇).

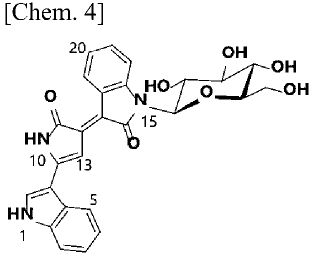

### 5. Physical Properties of Compound (I)

The physicochemical properties of the compound (I) were as follows.

### (1) Ultraviolet-Visible Absorption Spectrum

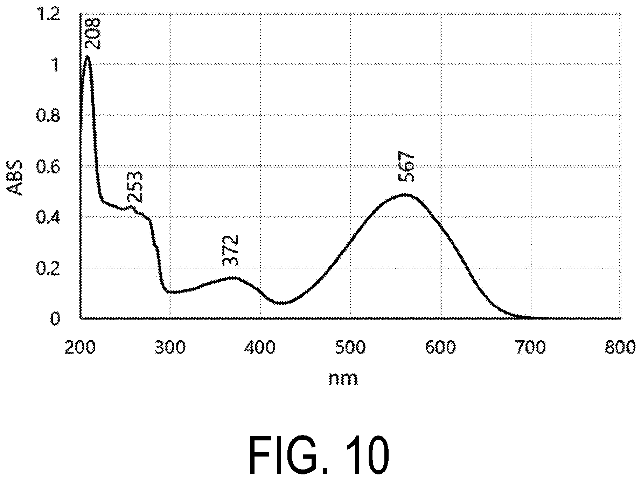

The ultraviolet-visible absorption spectrum was measured at 25°C using a UV-visible spectrophotometer V-650 (manufactured by JASCO Corporation) with a quartz cell having an optical path length of 0.1 mm. The results are shown in FIG. 10.

### (2) Molecular Formula Determination by Liquid Chromatography-Mass Spectrometry (LC-MS)

LC-MS analysis was performed in the positive ion mode using a system comprising an LCMS-IT-TOF mass spectrometer, Prominence HPLC, and LCMSsolution software version 3.81.

The column used was a Shim-pack FC-ODS column (manufactured by Shimadzu Corporation) having dimensions of 2.0 mm inner diameter × 150 mm length. The mobile phase consisted of (A) a 0.1% aqueous trifluoroacetic acid solution and (B) a 90% aqueous acetonitrile solution containing 0.1% trifluoroacetic acid. Gradient elution was carried out under the following conditions: solvent B, 20% hold (0 to 0.04 min), 20% to 70% (0.04 to 10 min), 70% hold (10 to 16 min), 70% to 20% (16 to 17 min), and 20% hold (17 to 28 min).

The flow rate was set to 0.4 mL/min, and the column oven temperature was maintained at 40°C. When 10 µL of a methanol extract of petals was subjected to analysis, a peak of Compound (I) having absorbance at 570 nm was observed at a retention time of 5.3 minutes.

Based on analysis of the mass spectrum, the molecular formula was determined as follows: HRMS (ESI, *m*/*z*, [M+H]⁺) Calcd for C₂₆H₂₃N₃O₇: 490.1609; Found: 490.1595 (error -2.86 ppm).

### (3) Circular Dichroism Spectrum

The circular dichroism spectrum was measured at 25°C using a J-1500 circular dichroism spectropolarimeter (manufactured by JASCO Corporation) with a quartz cell having an optical path length of 0.1 mm (results not shown).

### (4) Optical Rotation

Optical rotation was measured at 25°C using a DIP-1000 polarimeter (manufactured by JASCO Corporation) with a cylindrical quartz cell having dimensions of 3.5 mm in diameter × 100 mm in length (results not shown).

### (5) NMR Spectra

After dissolving 1.5 mg of Compound (I) in 0.65 mL of DMSO-*d*₆, NMR measurements were performed using an AVANCE III HD 800 spectrometer (manufactured by Bruker BioSpin).

One-dimensional ¹H and ¹³C NMR, and two-dimensional ¹H DQF-COSY, ¹H TOCSY, ¹H NOESY, ¹H ROESY, ¹H{¹³C} edited-HSQC, ¹H{¹³C} HMBC, ¹H{¹³C}-HMQC-COSY, and ¹H{¹⁵N}-HMBC measurements were carried out.

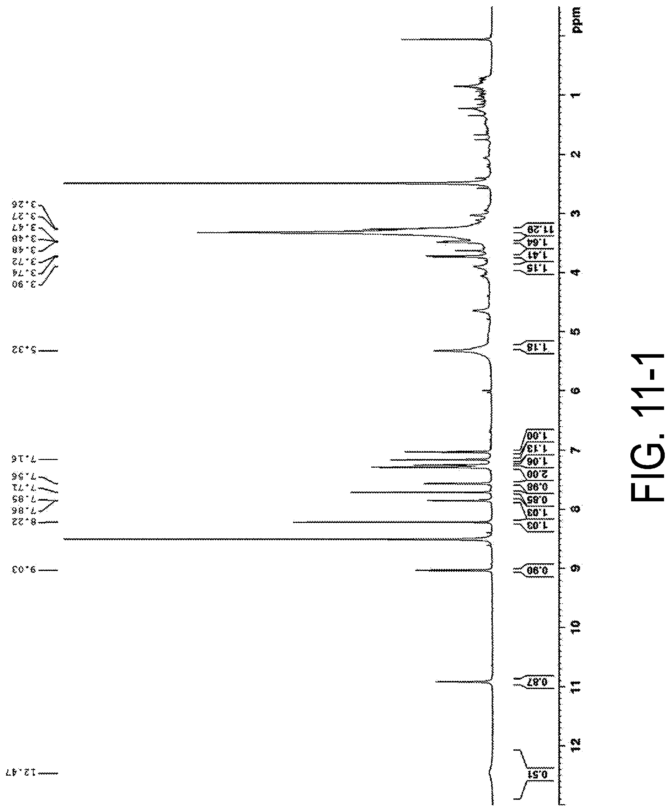

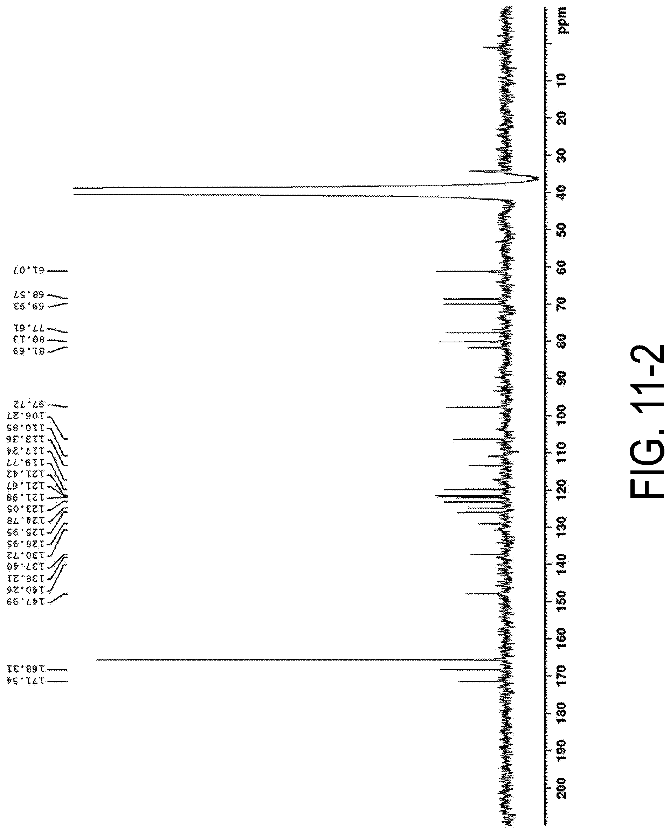

The respective results are shown in FIGS. 11-1 and 11-2.

### 6. Preparative HPLC of Compound (II)

Compound (II), which was eluted at retention times of 34.5 to 35.7 minutes during HPLC purification of the 60% methanol (MeOH) fraction obtained by Sep-Pak purification, was combined with the 80% MeOH fraction and concentrated. As a precipitate was formed, methanol was added until the precipitate was completely dissolved.

This solution was divided into ten portions of 3.2 mL each and subjected to purification using the above-described preparative HPLC system for blue pigments under the following gradient elution conditions: solvent B, 30% to 50% (0 to 0.05 min), 50% hold (0.05 to 26 min), 50% to 100% (26 to 27 min), 100% to 100% (27 to 28 min), 100% to 20% (28 to 29 min), and 20% hold (29 to 38 min).

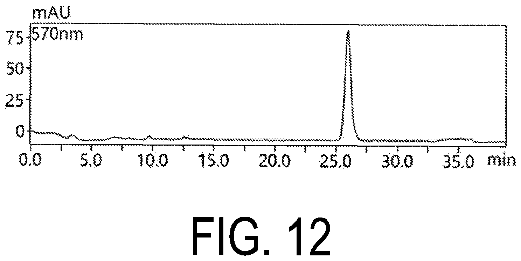

Fractions eluted at retention times of 25.6 to 26.8 minutes in the chromatogram shown in FIG. 12 were collected, and the organic solvent was removed using a SpeedVac concentrator, followed by lyophilization.

Compound (II) was obtained as a blue amorphous solid in an amount of 1.8 mg, corresponding to a yield of 2.9 × 10⁻³%.

The structure of this compound was determined by NMR, LC-MS, and the like to be represented by the following formula (II) (C₂₀H₁₃N₃O).

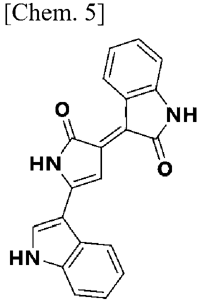

### 7. Physical Properties of Compound (II)

The physicochemical properties of the compound (II) were as follows.

### (1) Ultraviolet-Visible Absorption Spectrum

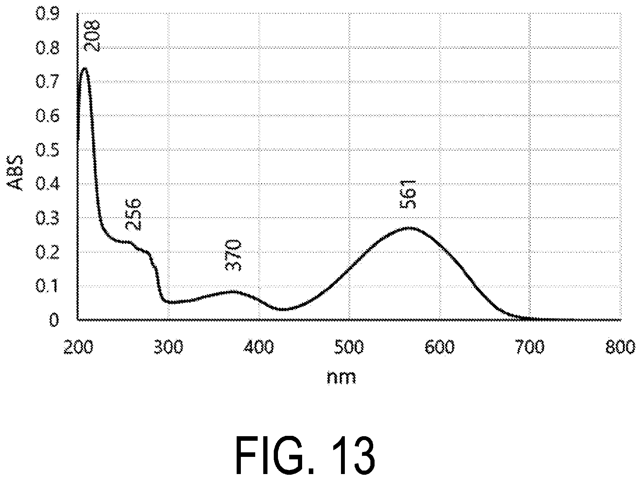

The ultraviolet-visible absorption spectrum was measured at 25°C using a UV-visible spectrophotometer V-650 (manufactured by JASCO Corporation) with a quartz cell having an optical path length of 0.1 mm. The results are shown in FIG. 13.

### (2) Molecular Formula Determination by Liquid Chromatography-Mass Spectrometry (LC-MS)

LC-MS analysis was performed using a system comprising an LCMS-IT-TOF mass spectrometer, Prominence HPLC, and LCMSsolution software (version 3.81).

The column used was a Shim-pack FC-ODS column (manufactured by Shimadzu Corporation) having dimensions of 2.0 mm inner diameter × 150 mm length. The mobile phase consisted of (A) a 0.1% aqueous trifluoroacetic acid solution and (B) a 90% aqueous acetonitrile solution containing 0.1% trifluoroacetic acid. Gradient elution was carried out under the following conditions: solvent B, 20% hold (0 to 0.04 min), 20% to 70% (0.04 to 10 min), 70% hold (10 to 16 min), 70% to 20% (16 to 17 min), and 20% hold (17 to 28 min).

The flow rate was set to 0.4 mL/min, and the column oven temperature was maintained at 40°C. When 10 µL of a fraction containing Compound (II) obtained by HPLC purification was subjected to analysis without concentration, a peak of Compound (II) was observed at a retention time of 8.6 minutes.

Based on analysis of the mass spectrum, the molecular formula was determined as follows: HRMS (ESI, m/z, [M+H]⁺) Calcd for C₂₀H₁₃N₃O: 328.1081; Found: 328.1091 (error +3.05 ppm).

### (3) Circular Dichroism Spectrum

The circular dichroism spectrum was measured at 25°C using a J-1500 circular dichroism spectropolarimeter (manufactured by JASCO Corporation) with a quartz cell having an optical path length of 0.1 mm (results not shown).

### (4) Optical Rotation

Optical rotation was measured at 25°C using a DIP-1000 polarimeter (manufactured by JASCO Corporation) with a cylindrical quartz cell having dimensions of 3.5 mm in diameter × 100 mm in length (results not shown).

### (5) NMR Spectra

After dissolving 1.8 mg of Compound (II) in DMSO-d₆, NMR measurements were performed using an AVANCE III HD 800 spectrometer (manufactured by Bruker BioSpin).

One-dimensional ¹H and ¹³C NMR, and two-dimensional ¹H DQF-COSY, ¹H TOCSY, ¹H NOESY, ¹H ROESY, ¹H{¹³C} edited-HSQC, ¹H{¹³C} HMBC, ¹H{¹³C}-HMQC-COSY, and ¹H{¹⁵N}-HMBC measurements were carried out.

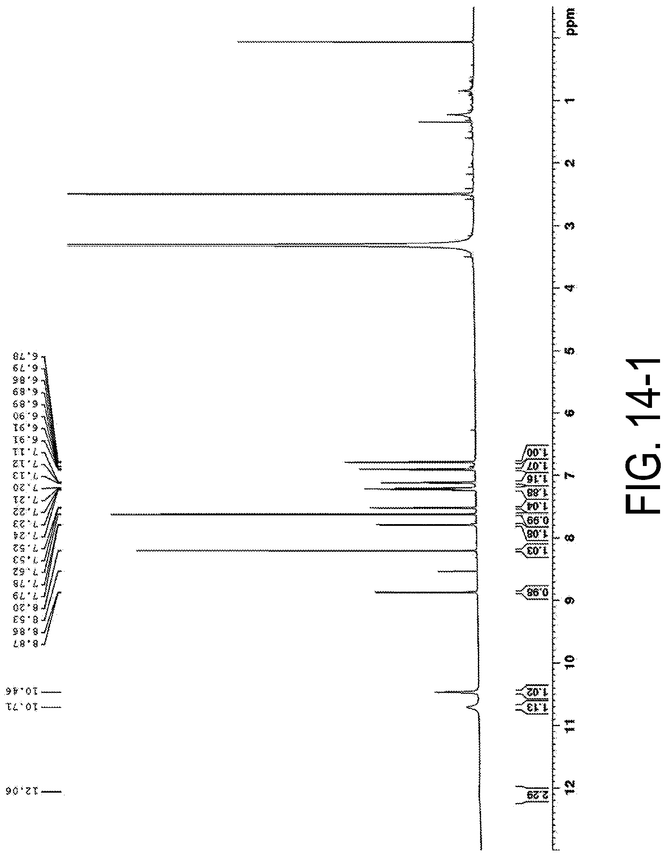

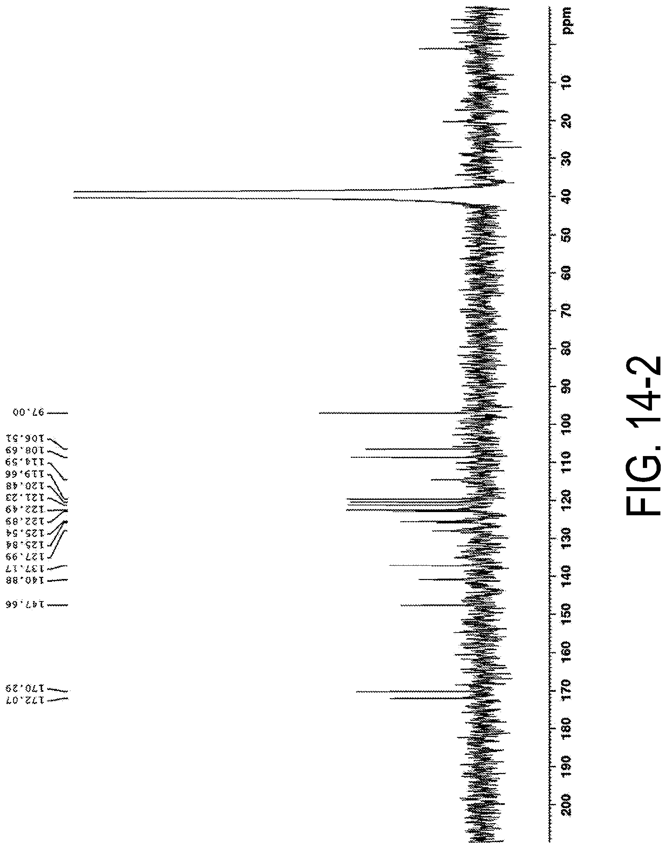

The respective results are shown in FIGS. 14-1 and 14-2.

### [Example 6: Acquisition of Rose Transgenic Plant with Deoxyviolacein Synthetic Gene Cluster Introduced]

The pSPB7673 or pSPB7674 produced in Example 4 was introduced into a blue categoly variety "Ocean Song", and the obtained rose transgenic callus was subjected to a regeneration treatment while being continuously maintained and propagated to obtain rose transgenic plants. The rose transgenic plants were cultivated in a greenhouse, and flowering individuals were evaluated and analyzed.

Pigment analysis of the petals was performed by HPLC (Shimadzu, prominence LC-20AD) under the conditions described in Example 2, and it was confirmed that the rose transgenic plants accumulated deoxyviolacein, the target component, as a main pigment and also accumulated the glycoside described in Example 5. The amount of deoxyviolacein accumulated was 0.102 mg at the maximum per gram of fresh petal weight.

The color of each petal was evaluated using a spectrophotometer (Minolta CM-700d). In the line in which deoxyviolacein was accumulated in petals, it was confirmed that the hue angle (Hue) was greatly shifted to the blue direction, and the minimum value (λMin) of the reflection spectrum was also shifted to the long wavelength side. In the visual evaluation, it was confirmed that the petals of the host plant were light pink, corresponding to 75C (Purple Group) in the RHS (Royal Horticultural Society) color chart, whereas the petals of the rose transgenic plants accumulating deoxyviolacein as the main pigment had changed to dark blue, among which the individual exhibiting the most pronounced bluish change showed a flower color corresponding to 96B and C (Violet-Blue Group) in the RHS color chart. The results showed that the color of petals changed to blue due to deoxyviolacein.

The analytical and colorimetric values of representative rose transgenic plants are presented in the table below.

**[Table 1]**

| code | Colorimetric value | Anthocyanidin | Flavonol | Violaceins | | | | | |
|---|---|---|---|---|---|---|---|---|---|
| | Hue (°) | Ref% (%) | λMin (nm) | Cya (mg/g) | Q (mg/g) | K (mg/g) | VC (mg/g) | Dvc (mg/g) | DvcG (mg/g) |
| OS cont. | 351.79 | 38.87 | 540 | 0.032 | 2.666 | 0.173 | 0 | 0 | 0 |
| OS/7673-53-04 | 295.55 | 12.88 | 570 | 0.004 | 3.060 | 1.482 | 0 | 0.102 | 0.004 |
| OS/7674-13-05 | 314.61 | 27.67 | 570 | 0.007 | 3.054 | 0.821 | 0 | 0.002 | 0.008 |
| OS/7674-65-02 | 283.30 | 18.21 | 580 | 0.004 | 2.469 | 1.208 | 0 | 0.019 | 0 |
| OS/7674-65-03 | 286.72 | 26.90 | 570 | 0.004 | 2.083 | 1.011 | 0 | 0.017 | 0.002 |
| OS/7674-71-02 | 318.26 | 24.21 | 570 | 0.004 | 2.059 | 1.323 | 0 | 0.19 | 0.004 |

| | | | | | | | | | |
|---|---|---|---|---|---|---|---|---|---|
| Hue: hue angle, Ref%: reflectance, λMin: minimum value of reflection spectrum, Cya: cyanidin, Q: quercetin, K: kaempferol, VC: violacein, Dvc: deoxyviolacein, DvcG: deoxyviolacein glycoside | | | | | | | | | |

## Claims

1. A VioA gene selected from the group consisting of:
(1-a) a polynucleotide consisting of a nucleotide sequence of SEQ ID NO: 1;
(1-b) a polynucleotide that hybridizes under stringent conditions with another polynucleotide consisting of a nucleotide sequence complementary to the nucleotide sequence of SEQ ID NO: 1, wherein the polynucleotide encodes a protein having a function of converting L-tryptophan into indole-3-pyruvic acid imine (IPA imine);
(1-c) a polynucleotide that encodes a protein consisting of an amino acid sequence of SEQ ID NO: 2;
(1-d) a polynucleotide that encodes a protein consisting of an amino acid sequence in which one or several amino acids are deleted, substituted, inserted, and/or added in the amino acid sequence of SEQ ID NO: 2, the protein having a function of converting L-tryptophan into indole-3-pyruvic acid imine (IPA imine); and
(1-e) a polynucleotide that encodes a protein having an amino acid sequence with 90% or more identity to the amino acid sequence of SEQ ID NO: 2, the protein having a function of converting L-tryptophan into indole-3-pyruvic acid imine (IPA imine).

2. A VioB gene selected from the group consisting of:
(2-a) a polynucleotide consisting of a nucleotide sequence of SEQ ID NO: 3;
(2-b) a polynucleotide that hybridizes under stringent conditions with another polynucleotide consisting of a nucleotide sequence complementary to the nucleotide sequence of SEQ ID NO: 3, wherein the polynucleotide encodes a protein having a function of converting IPA imine into an IPA imine dimer;
(2-c) a polynucleotide that encodes a protein consisting of an amino acid sequence of SEQ ID NO: 4;
(2-d) a polynucleotide that encodes a protein consisting of an amino acid sequence in which one or several amino acids are deleted, substituted, inserted, and/or added in the amino acid sequence of SEQ ID NO: 4, the protein having a function of converting IPA imine into an IPA imine dimer; and
(2-e) a polynucleotide that encodes a protein having an amino acid sequence with 90% or more identity to the amino acid sequence of SEQ ID NO: 4, the protein having a function of converting IPA imine into an IPA imine dimer.

3. A VioC gene selected from the group consisting of:
(3-a) a polynucleotide consisting of a nucleotide sequence of SEQ ID NO: 5;
(3-b) a polynucleotide that hybridizes under stringent conditions with another polynucleotide consisting of a nucleotide sequence complementary to the nucleotide sequence of SEQ ID NO: 5, wherein the polynucleotide encodes a protein having a function of converting prodeoxyviolaceinic acid into deoxyviolaceinic acid;
(3-c) a polynucleotide that encodes a protein consisting of an amino acid sequence of SEQ ID NO: 6;
(3-d) a polynucleotide that encodes a protein consisting of an amino acid sequence in which one or several amino acids are deleted, substituted, inserted, and/or added in the amino acid sequence of SEQ ID NO: 6, the protein having a function of converting prodeoxyviolaceinic acid into deoxyviolaceinic acid; and
(3-e) a polynucleotide that encodes a protein having an amino acid sequence with 90% or more identity to the amino acid sequence of SEQ ID NO: 6, the protein having a function of converting prodeoxyviolaceinic acid into deoxyviolaceinic acid.

4. A VioE gene selected from the group consisting of:
(4-a) a polynucleotide consisting of a nucleotide sequence of SEQ ID NO: 7;
(4-b) a polynucleotide that hybridizes under stringent conditions with another polynucleotide consisting of a nucleotide sequence complementary to the nucleotide sequence of SEQ ID NO: 7, wherein the polynucleotide encodes a protein having a function of converting an IPA imine dimer into prodeoxyviolaceinic acid;
(4-c) a polynucleotide that encodes a protein consisting of an amino acid sequence of SEQ ID NO: 8;
(4-d) a polynucleotide that encodes a protein, the protein consisting of an amino acid sequence in which one or several amino acids are deleted, substituted, inserted, and/or added in the amino acid sequence of SEQ ID NO: 8, the protein having a function of converting an IPA imine dimer into prodeoxyviolaceinic acid; and
(4-e) a polynucleotide that encodes a protein, the protein having an amino acid sequence with 90% or more identity to the amino acid sequence of SEQ ID NO: 8, the protein having a function of converting an IPA imine dimer into prodeoxyviolaceinic acid.

5. A vector, comprising a VioA gene, a VioB gene, a VioC gene, a VioE gene, or any combination thereof, wherein
the VioA gene is selected from the group consisting of:
(1-a) a polynucleotide consisting of a nucleotide sequence of SEQ ID NO: 1;
(1-b) a polynucleotide that hybridizes under stringent conditions with another polynucleotide consisting of a nucleotide sequence complementary to the nucleotide sequence of SEQ ID NO: 1, wherein the polynucleotide encodes a protein having a function of converting L-tryptophan into indole-3-pyruvic acid imine (IPA imine);
(1-c) a polynucleotide that encodes a protein consisting of an amino acid sequence of SEQ ID NO: 2;
(1-d) a polynucleotide that encodes a protein, the protein consisting of an amino acid sequence in which one or several amino acids are deleted, substituted, inserted, and/or added in the amino acid sequence of SEQ ID NO: 2, the protein having a function of converting L-tryptophan into indole-3-pyruvic acid imine (IPA imine); and
(1-e) a polynucleotide that encodes a protein having an amino acid sequence with 90% or more identity to the amino acid sequence of SEQ ID NO: 2, the protein having a function of converting L-tryptophan into indole-3-pyruvic acid imine (IPA imine),
the VioB gene is selected from the group consisting of:
(2-a) a polynucleotide consisting of a nucleotide sequence of SEQ ID NO: 3;
(2-b) a polynucleotide that hybridizes under stringent conditions with another polynucleotide consisting of a nucleotide sequence complementary to the nucleotide sequence of SEQ ID NO: 3, wherein the polynucleotide encodes a protein having a function of converting IPA imine into an IPA imine dimer;
(2-c) a polynucleotide that encodes a protein consisting of an amino acid sequence of SEQ ID NO: 4;
(2-d) a polynucleotide that encodes a protein consisting of an amino acid sequence in which one or several amino acids are deleted, substituted, inserted, and/or added in the amino acid sequence of SEQ ID NO: 4, the protein having a function of converting IPA imine into an IPA imine dimer; and
(2-e) a polynucleotide that encodes a protein having an amino acid sequence with 90% or more identity to the amino acid sequence of SEQ ID NO: 4, the protein having a function of converting IPA imine into an IPA imine dimer,
the VioC gene is selected from the group consisting of:
(3-a) a polynucleotide consisting of a nucleotide sequence of SEQ ID NO: 5;
(3-b) a polynucleotide that hybridizes under stringent conditions with another polynucleotide consisting of a nucleotide sequence complementary to the nucleotide sequence of SEQ ID NO: 5, wherein the polynucleotide encodes a protein having a function of converting prodeoxyviolaceinic acid into deoxyviolaceinic acid;
(3-c) a polynucleotide that encodes a protein consisting of an amino acid sequence of SEQ ID NO: 6;
(3-d) a polynucleotide that encodes a protein consisting of an amino acid sequence in which one or several amino acids are deleted, substituted, inserted, and/or added in the amino acid sequence of SEQ ID NO: 6, the protein having a function of converting prodeoxyviolaceinic acid into deoxyviolaceinic acid; and
(3-e) a polynucleotide that encodes a protein having an amino acid sequence with 90% or more identity to the amino acid sequence of SEQ ID NO: 6, the protein having a function of converting prodeoxyviolaceinic acid into deoxyviolaceinic acid, and
the VioE gene is selected from the group consisting of:
(4-a) a polynucleotide consisting of a nucleotide sequence of SEQ ID NO: 7;
(4-b) a polynucleotide that hybridizes under stringent conditions with another polynucleotide consisting of a nucleotide sequence complementary to the nucleotide sequence of SEQ ID NO: 7, wherein the polynucleotide encodes a protein having a function of converting an IPA imine dimer into prodeoxyviolaceinic acid;
(4-c) a polynucleotide that encodes a protein consisting of an amino acid sequence of SEQ ID NO: 8;
(4-d) a polynucleotide that encodes a protein consisting of an amino acid sequence in which one or several amino acids are deleted, substituted, inserted, and/or added in the amino acid sequence of SEQ ID NO: 8, the protein having a function of converting an IPA imine dimer into prodeoxyviolaceinic acid; and
(4-e) a polynucleotide that encodes a protein having an amino acid sequence with 90% or more identity to the amino acid sequence of SEQ ID NO: 8, the protein having a function of converting an IPA imine dimer into prodeoxyviolaceinic acid.

6. The vector according to claim 5, further comprising the VioA gene and a promoter linked to the VioA gene and configured to function as a petal-specific promoter, wherein the promoter is selected from a lisianthus-derived anthocyanidin synthase gene promoter (SEQ ID NO: 9), a morning glory-derived dihydroflavonol 4-reductase gene promoter (SEQ ID NO: 10), or a rose-derived chalcone synthase gene promoter (SEQ ID NO: 11).

7. The vector according to claim 5 or 6, further comprising an untranslated region (5'-UTR) derived from Arabidopsis thaliana-derived alcohol dehydrogenase (ADH) gene (SEQ ID NO: 12) operably linked to the VioA gene, the VioB gene, the VioC gene, and/or the VioE gene.

8. A transgenic plant or its inbred or outbred progeny, comprising a VioA gene, a VioB gene, a VioC gene, a VioE gene, or any combination thereof, wherein
the VioA gene is selected from the group consisting of:
(1-a) a polynucleotide consisting of a nucleotide sequence of SEQ ID NO: 1;
(1-b) a polynucleotide that hybridizes under stringent conditions with another polynucleotide consisting of a nucleotide sequence complementary to the nucleotide sequence of SEQ ID NO: 1, wherein the polynucleotide encodes a protein having a function of converting L-tryptophan into indole-3-pyruvic acid imine (IPA imine);
(1-c) a polynucleotide that encodes a protein consisting of an amino acid sequence of SEQ ID NO: 2;
(1-d) a polynucleotide that encodes a protein consisting of an amino acid sequence in which one or several amino acids are deleted, substituted, inserted, and/or added in the amino acid sequence of SEQ ID NO: 2, the protein having a function of converting L-tryptophan into indole-3-pyruvic acid imine (IPA imine); and
(1-e) a polynucleotide that encodes a protein having an amino acid sequence with 90% or more identity to the amino acid sequence of SEQ ID NO: 2, the protein having a function of converting L-tryptophan into indole-3-pyruvic acid imine (IPA imine),
the VioB gene is selected from the group consisting of:
(2-a) a polynucleotide consisting of a nucleotide sequence of SEQ ID NO: 3;
(2-b) a polynucleotide that hybridizes under stringent conditions with another polynucleotide consisting of a nucleotide sequence complementary to the nucleotide sequence of SEQ ID NO: 3, wherein the polynucleotide encodes a protein having a function of converting IPA imine into an IPA imine dimer;
(2-c) a polynucleotide that encodes a protein consisting of an amino acid sequence of SEQ ID NO: 4;
(2-d) a polynucleotide that encodes a protein consisting of an amino acid sequence in which one or several amino acids are deleted, substituted, inserted, and/or added in the amino acid sequence of SEQ ID NO: 4, the protein having a function of converting IPA imine into an IPA imine dimer; and
(2-e) a polynucleotide that encodes a protein having an amino acid sequence with 90% or more identity to the amino acid sequence of SEQ ID NO: 4, the protein having a function of converting IPA imine into an IPA imine dimer,
the VioC gene is selected from the group consisting of:
(3-a) a polynucleotide consisting of a nucleotide sequence of SEQ ID NO: 5;
(3-b) a polynucleotide that hybridizes under stringent conditions with another polynucleotide consisting of a nucleotide sequence complementary to the nucleotide sequence of SEQ ID NO: 5, wherein the polynucleotide encodes a protein having a function of converting prodeoxyviolaceinic acid into deoxyviolaceinic acid;
(3-c) a polynucleotide that encodes a protein consisting of an amino acid sequence of SEQ ID NO: 6;
(3-d) a polynucleotide that encodes a protein consisting of an amino acid sequence in which one or several amino acids are deleted, substituted, inserted, and/or added in the amino acid sequence of SEQ ID NO: 6, the protein having a function of converting prodeoxyviolaceinic acid into deoxyviolaceinic acid; and
(3-e) a polynucleotide that encodes a protein having an amino acid sequence with 90% or more identity to the amino acid sequence of SEQ ID NO: 6, the protein having a function of converting prodeoxyviolaceinic acid into deoxyviolaceinic acid, and
the VioE gene is selected from the group consisting of:
(4-a) a polynucleotide consisting of a nucleotide sequence of SEQ ID NO: 7;
(4-b) a polynucleotide that hybridizes under stringent conditions with another polynucleotide consisting of a nucleotide sequence complementary to the nucleotide sequence of SEQ ID NO: 7, wherein the polynucleotide encodes a protein having a function of converting an IPA imine dimer into prodeoxyviolaceinic acid;
(4-c) a polynucleotide that encodes a protein consisting of an amino acid sequence of SEQ ID NO: 8;
(4-d) a polynucleotide that encodes a protein consisting of an amino acid sequence in which one or several amino acids are deleted, substituted, inserted, and/or added in the amino acid sequence of SEQ ID NO: 8, the protein having a function of converting an IPA imine dimer into prodeoxyviolaceinic acid; and
(4-e) a polynucleotide that encodes a protein having an amino acid sequence with 90% or more identity to the amino acid sequence of SEQ ID NO: 8, the protein having a function of converting an IPA imine dimer into prodeoxyviolaceinic acid.

9. The transgenic plant or its inbred or outbred progeny according to claim 8, wherein the plant is a rose, carnation, chrysanthemum, or lily.

10. A propagule, partial plant body, tissue, or cell of the transgenic plant or its inbred or outbred progeny according to claim 8 or 9.

11. A cut flower of the transgenic plant or its inbred or outbred progeny according to claim 8 or 9, or a processed form created from the cut flower.

12. A method for producing a transgenic plant, the method comprising introducing the gene according to any one of claims 1 to 4 or the vector according to claim 5 into a cell of a plant containing L-tryptophan.

13. The method according to claim 12, wherein the plant is a rose, carnation, chrysanthemum, or lily.

14. A compound represented by a formula set forth below: 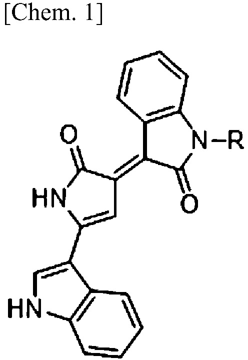 where in the formula, R is a monosaccharide unit selected from glucose, mannose, galactose, fucose, rhamnose, arabinose, xylose, fructose, or glucuronic acid, or a sugar chain formed by linking two or more of the monosaccharide units.

15. The compound according to claim 14, wherein R in the formula is glucose.

16. A dye, comprising the compound according to claim 14 or 15.

17. A textile product, comprising the dye according to claim 16.

18. A colorant, comprising the compound according to claim 14 or 15.

19. A medicament, cosmetic, food, or beverage, comprising the compound according to claim 14 or 15.

20. A method for producing deoxyviolacein and/or a glycoside thereof, the method comprising:
introducing the gene according to any one of claims 1 to 4 or the vector according to claim 5 into a cell of a plant containing L-tryptophan; and
collecting the compound from the cell.

21. The method according to claim 20, wherein the glycoside is the compound according to claim 14 or 15.

22. The method according to claim 20, wherein the plant is a rose, carnation, chrysanthemum, or lily.

23. A plant or its inbred or outbred progeny, comprising deoxyviolacein and/or a glycoside thereof.

24. The plant or its inbred or outbred progeny according to claim 23, wherein the glycoside is the compound according to claim 14 or 15.

25. The plant or its inbred or outbred progeny according to claim 23, wherein the plant is a rose, carnation, chrysanthemum, or lily.

26. A propagule, partial plant body, tissue, or cell of the plant or its inbred or outbred progeny according to claim 23.

27. A cut flower of the plant or its inbred or outbred progeny according to claim 23, or a processed form created from the cut flower.

## Amended claims

### Amended claims under Art. 19.1 PCT

1. A Vio gene, comprising a codon optimized based on rose genomic information, the Vio gene being a VioA gene selected from the group consisting of:
(1-a) a polynucleotide consisting of a nucleotide sequence of SEQ ID NO: 1;
(1-b) a polynucleotide that hybridizes under stringent conditions with another polynucleotide consisting of a nucleotide sequence complementary to the nucleotide sequence of SEQ ID NO: 1, wherein the polynucleotide encodes a protein having a function of converting L-tryptophan into indole-3-pyruvic acid imine (IPA imine);
(1-c) a polynucleotide that encodes a protein consisting of an amino acid sequence of SEQ ID NO: 2;
(1-d) a polynucleotide that encodes a protein consisting of an amino acid sequence in which one or several amino acids are deleted, substituted, inserted, and/or added in the amino acid sequence of SEQ ID NO: 2, the protein having a function of converting L-tryptophan into indole-3-pyruvic acid imine (IPA imine); and
(1-e) a polynucleotide that encodes a protein having an amino acid sequence with 90% or more identity to the amino acid sequence of SEQ ID NO: 2, the protein having a function of converting L-tryptophan into indole-3-pyruvic acid imine (IPA imine).

2. A Vio gene, comprising a codon optimized based on rose genomic information, the Vio gene being a VioB gene selected from the group consisting of:
(2-a) a polynucleotide consisting of a nucleotide sequence of SEQ ID NO: 3;
(2-b) a polynucleotide that hybridizes under stringent conditions with another polynucleotide consisting of a nucleotide sequence complementary to the nucleotide sequence of SEQ ID NO: 3, wherein the polynucleotide encodes a protein having a function of converting IPA imine into an IPA imine dimer;
(2-c) a polynucleotide that encodes a protein consisting of an amino acid sequence of SEQ ID NO: 4;
(2-d) a polynucleotide that encodes a protein consisting of an amino acid sequence in which one or several amino acids are deleted, substituted, inserted, and/or added in the amino acid sequence of SEQ ID NO: 4, the protein having a function of converting IPA imine into an IPA imine dimer; and
(2-e) a polynucleotide that encodes a protein having an amino acid sequence with 90% or more identity to the amino acid sequence of SEQ ID NO: 4, the protein having a function of converting IPA imine into an IPA imine dimer.

3. A Vio gene, comprising a codon optimized based on rose genomic information, the Vio gene being a VioC gene selected from the group consisting of:
(3-a) a polynucleotide consisting of a nucleotide sequence of SEQ ID NO: 5;
(3-b) a polynucleotide that hybridizes under stringent conditions with another polynucleotide consisting of a nucleotide sequence complementary to the nucleotide sequence of SEQ ID NO: 5, wherein the polynucleotide encodes a protein having a function of converting prodeoxyviolaceinic acid into deoxyviolaceinic acid;
(3-c) a polynucleotide that encodes a protein consisting of an amino acid sequence of SEQ ID NO: 6;
(3-d) a polynucleotide that encodes a protein consisting of an amino acid sequence in which one or several amino acids are deleted, substituted, inserted, and/or added in the amino acid sequence of SEQ ID NO: 6, the protein having a function of converting prodeoxyviolaceinic acid into deoxyviolaceinic acid; and
(3-e) a polynucleotide that encodes a protein having an amino acid sequence with 90% or more identity to the amino acid sequence of SEQ ID NO: 6, the protein having a function of converting prodeoxyviolaceinic acid into deoxyviolaceinic acid.

4. A Vio gene, comprising a codon optimized based on rose genomic information, the Vio gene being a VioE gene selected from the group consisting of:
(4-a) a polynucleotide consisting of a nucleotide sequence of SEQ ID NO: 7;
(4-b) a polynucleotide that hybridizes under stringent conditions with another polynucleotide consisting of a nucleotide sequence complementary to the nucleotide sequence of SEQ ID NO: 7, wherein the polynucleotide encodes a protein having a function of converting an IPA imine dimer into prodeoxyviolaceinic acid;
(4-c) a polynucleotide that encodes a protein consisting of an amino acid sequence of SEQ ID NO: 8;
(4-d) a polynucleotide that encodes a protein consisting of an amino acid sequence in which one or several amino acids are deleted, substituted, inserted, and/or added in the amino acid sequence of SEQ ID NO: 8, the protein having a function of converting an IPA imine dimer into prodeoxyviolaceinic acid; and
(4-e) a polynucleotide that encodes a protein having an amino acid sequence with 90% or more identity to the amino acid sequence of SEQ ID NO: 8, the protein having a function of converting an IPA imine dimer into prodeoxyviolaceinic acid.

5. A vector, comprising a Vio gene comprising a codon optimized based on rose genomic information, the Vio gene comprising a VioA gene, a VioB gene, a VioC gene, a VioE gene, or any combination thereof, wherein
the VioA gene is selected from the group consisting of:
(1-a) a polynucleotide consisting of a nucleotide sequence of SEQ ID NO: 1;
(1-b) a polynucleotide that hybridizes under stringent conditions with another polynucleotide consisting of a nucleotide sequence complementary to the nucleotide sequence of SEQ ID NO: 1, wherein the polynucleotide encodes a protein having a function of converting L-tryptophan into indole-3-pyruvic acid imine (IPA imine);
(1-c) a polynucleotide that encodes a protein consisting of an amino acid sequence of SEQ ID NO: 2;
(1-d) a polynucleotide that encodes a protein consisting of an amino acid sequence in which one or several amino acids are deleted, substituted, inserted, and/or added in the amino acid sequence of SEQ ID NO: 2, the protein having a function of converting L-tryptophan into indole-3-pyruvic acid imine (IPA imine); and
(1-e) a polynucleotide that encodes a protein having an amino acid sequence with 90% or more identity to the amino acid sequence of SEQ ID NO: 2, the protein having a function of converting L-tryptophan into indole-3-pyruvic acid imine (IPA imine),
the VioB gene is selected from the group consisting of:
(2-a) a polynucleotide consisting of a nucleotide sequence of SEQ ID NO: 3;
(2-b) a polynucleotide that hybridizes under stringent conditions with another polynucleotide consisting of a nucleotide sequence complementary to the nucleotide sequence of SEQ ID NO: 3, wherein the polynucleotide encodes a protein having a function of converting IPA imine into an IPA imine dimer;
(2-c) a polynucleotide that encodes a protein consisting of an amino acid sequence of SEQ ID NO: 4;
(2-d) a polynucleotide that encodes a protein consisting of an amino acid sequence in which one or several amino acids are deleted, substituted, inserted, and/or added in the amino acid sequence of SEQ ID NO: 4, the protein having a function of converting IPA imine into an IPA imine dimer; and
(2-e) a polynucleotide that encodes a protein having an amino acid sequence with 90% or more identity to the amino acid sequence of SEQ ID NO: 4, the protein having a function of converting IPA imine into an IPA imine dimer,
the VioC gene is selected from the group consisting of:
(3-a) a polynucleotide consisting of a nucleotide sequence of SEQ ID NO: 5;
(3-b) a polynucleotide that hybridizes under stringent conditions with another polynucleotide consisting of a nucleotide sequence complementary to the nucleotide sequence of SEQ ID NO: 5, wherein the polynucleotide encodes a protein having a function of converting prodeoxyviolaceinic acid into deoxyviolaceinic acid;
(3-c) a polynucleotide that encodes a protein consisting of an amino acid sequence of SEQ ID NO: 6;
(3-d) a polynucleotide that encodes a protein consisting of an amino acid sequence in which one or several amino acids are deleted, substituted, inserted, and/or added in the amino acid sequence of SEQ ID NO: 6, the protein having a function of converting prodeoxyviolaceinic acid into deoxyviolaceinic acid; and
(3-e) a polynucleotide that encodes a protein having an amino acid sequence with 90% or more identity to the amino acid sequence of SEQ ID NO: 6, the protein having a function of converting prodeoxyviolaceinic acid into deoxyviolaceinic acid, and
the VioE gene is selected from the group consisting of:
(4-a) a polynucleotide consisting of a nucleotide sequence of SEQ ID NO: 7;
(4-b) a polynucleotide that hybridizes under stringent conditions with another polynucleotide consisting of a nucleotide sequence complementary to the nucleotide sequence of SEQ ID NO: 7, wherein the polynucleotide encodes a protein having a function of converting an IPA imine dimer into prodeoxyviolaceinic acid;
(4-c) a polynucleotide that encodes a protein consisting of an amino acid sequence of SEQ ID NO: 8;
(4-d) a polynucleotide that encodes a protein consisting of an amino acid sequence in which one or several amino acids are deleted, substituted, inserted, and/or added in the amino acid sequence of SEQ ID NO: 8, the protein having a function of converting an IPA imine dimer into prodeoxyviolaceinic acid; and
(4-e) a polynucleotide that encodes a protein having an amino acid sequence with 90% or more identity to the amino acid sequence of SEQ ID NO: 8, the protein having a function of converting an IPA imine dimer into prodeoxyviolaceinic acid.

6. The vector according to claim 5, further comprising the VioA gene and a promoter linked to the VioA gene and configured to function as a petal-specific promoter, wherein the promoter is selected from a lisianthus-derived anthocyanidin synthase gene promoter(SEQ ID NO: 9), a morning glory-derived dihydroflavonol 4-reductase gene promoter (SEQ ID NO: 10), or a rose-derived chalcone synthase gene promoter (SEQ ID NO: 11).

7. The vector according to claim 5 or 6, further comprising an untranslated region (5'-UTR) derived from Arabidopsis thaliana-derived alcohol dehydrogenase (ADH) gene (SEQ ID NO: 12) operably linked to the VioA gene, the VioB gene, the VioC gene, and/or the VioE gene.

8. A transgenic plant or its inbred or outbred progeny, comprising a Vio gene comprising a codon optimized based on rose genomic information, the Vio gene comprising a VioA gene, a VioB gene, a VioC gene, a VioE gene, or any combination thereof, wherein
the VioA gene is selected from the group consisting of:
(1-a) a polynucleotide consisting of a nucleotide sequence of SEQ ID NO: 1;
(1-b) a polynucleotide that hybridizes under stringent conditions with another polynucleotide consisting of a nucleotide sequence complementary to the nucleotide sequence of SEQ ID NO: 1, wherein the polynucleotide encodes a protein having a function of converting L-tryptophan into indole-3-pyruvic acid imine (IPA imine);
(1-c) a polynucleotide that encodes a protein consisting of an amino acid sequence of SEQ ID NO: 2;
(1-d) a polynucleotide that encodes a protein consisting of an amino acid sequence in which one or several amino acids are deleted, substituted, inserted, and/or added in the amino acid sequence of SEQ ID NO: 2, the protein having a function of converting L-tryptophan into indole-3-pyruvic acid imine (IPA imine); and
(1-e) a polynucleotide that encodes a protein having an amino acid sequence with 90% or more identity to the amino acid sequence of SEQ ID NO: 2, the protein having a function of converting L-tryptophan into indole-3-pyruvic acid imine (IPA imine),
the VioB gene is selected from the group consisting of:
(2-a) a polynucleotide consisting of a nucleotide sequence of SEQ ID NO: 3;
(2-b) a polynucleotide that hybridizes under stringent conditions with another polynucleotide consisting of a nucleotide sequence complementary to the nucleotide sequence of SEQ ID NO: 3, wherein the polynucleotide encodes a protein having a function of converting IPA imine into an IPA imine dimer;
(2-c) a polynucleotide that encodes a protein consisting of an amino acid sequence of SEQ ID NO: 4;
(2-d) a polynucleotide that encodes a protein consisting of an amino acid sequence in which one or several amino acids are deleted, substituted, inserted, and/or added in the amino acid sequence of SEQ ID NO: 4, the protein having a function of converting IPA imine into an IPA imine dimer; and
(2-e) a polynucleotide that encodes a protein having an amino acid sequence with 90% or more identity to the amino acid sequence of SEQ ID NO: 4, the protein having a function of converting IPA imine into an IPA imine dimer,
the VioC gene is selected from the group consisting of:
(3-a) a polynucleotide consisting of a nucleotide sequence of SEQ ID NO: 5;
(3-b) a polynucleotide that hybridizes under stringent conditions with another polynucleotide consisting of a nucleotide sequence complementary to the nucleotide sequence of SEQ ID NO: 5, wherein the polynucleotide encodes a protein having a function of converting prodeoxyviolaceinic acid into deoxyviolaceinic acid;
(3-c) a polynucleotide that encodes a protein consisting of an amino acid sequence of SEQ ID NO: 6;
(3-d) a polynucleotide that encodes a protein consisting of an amino acid sequence in which one or several amino acids are deleted, substituted, inserted, and/or added in the amino acid sequence of SEQ ID NO: 6, the protein having a function of converting prodeoxyviolaceinic acid into deoxyviolaceinic acid; and
(3-e) a polynucleotide that encodes a protein having an amino acid sequence with 90% or more identity to the amino acid sequence of SEQ ID NO: 6, the protein having a function of converting prodeoxyviolaceinic acid into deoxyviolaceinic acid, and
the VioE gene is selected from the group consisting of:
(4-a) a polynucleotide consisting of a nucleotide sequence of SEQ ID NO: 7;
(4-b) a polynucleotide that hybridizes under stringent conditions with another polynucleotide consisting of a nucleotide sequence complementary to the nucleotide sequence of SEQ ID NO: 7, wherein the polynucleotide encodes a protein having a function of converting an IPA imine dimer into prodeoxyviolaceinic acid;
(4-c) a polynucleotide that encodes a protein consisting of an amino acid sequence of SEQ ID NO: 8;
(4-d) a polynucleotide that encodes a protein consisting of an amino acid sequence in which one or several amino acids are deleted, substituted, inserted, and/or added in the amino acid sequence of SEQ ID NO: 8, the protein having a function of converting an IPA imine dimer into prodeoxyviolaceinic acid; and
(4-e) a polynucleotide that encodes a protein having an amino acid sequence with 90% or more identity to the amino acid sequence of SEQ ID NO: 8, the protein having a function of converting an IPA imine dimer into prodeoxyviolaceinic acid.

9. The transgenic plant or its inbred or outbred progeny according to claim 8, wherein the plant is a rose, carnation, chrysanthemum, or lily.

10. A propagule, partial plant body, tissue, or cell of the transgenic plant or its inbred or outbred progeny according to claim 8 or 9.

11. A cut flower of the transgenic plant or its inbred or outbred progeny according to claim 8 or 9, or a processed form created from the cut flower.

12. A method for producing a transgenic plant, the method comprising introducing the gene according to any one of claims 1 to 4 or the vector according to claim 5 into a cell of a plant containing L-tryptophan.

13. The method according to claim 12, wherein the plant is a rose, carnation, chrysanthemum, or lily.

14. A compound represented by a formula set forth below: 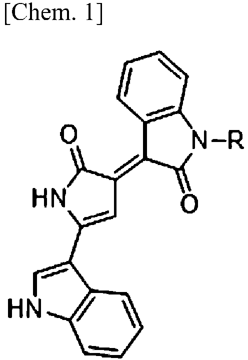 where in the formula, R is a monosaccharide unit selected from glucose, mannose, galactose, fucose, rhamnose, arabinose, xylose, fructose, or glucuronic acid, or a sugar chain formed by linking two or more of the monosaccharide units.

15. The compound according to claim 14, wherein R in the formula is glucose.

16. A dye, comprising the compound according to claim 14 or 15.

17. A textile product, comprising the dye according to claim 16.

18. A colorant, comprising the compound according to claim 14 or 15.

19. A medicament, cosmetic, food, or beverage, comprising the compound according to claim 14 or 15.

20. A method for producing deoxyviolacein and/or a glycoside thereof, the method comprising:
introducing the gene according to any one of claims 1 to 4 or the vector according to claim 5 into a cell of a plant containing L-tryptophan; and
collecting the compound from the cell.

21. The method according to claim 20, wherein the glycoside is the compound according to claim 14 or 15.

22. The method according to claim 20, wherein the plant is a rose, carnation, chrysanthemum, or lily.

23. A plant or its inbred or outbred progeny, comprising deoxyviolacein and/or a glycoside thereof.

24. The plant or its inbred or outbred progeny according to claim 23, wherein the glycoside is the compound according to claim 14 or 15.

25. The plant or its inbred or outbred progeny according to claim 23, wherein the plant is a rose, carnation, chrysanthemum, or lily.

26. A propagule, partial plant body, tissue, or cell of the plant or its inbred or outbred progeny according to claim 23.

27. A cut flower of the plant or its inbred or outbred progeny according to claim 23, or a processed form created from the cut flower.

Statement under Art. 19.1 PCT
1. Contents of Amendment
 ased on the description in, for example, paragraph 0014 of the specification of the subject patent application, the phrase "Vio gene comprising a codon optimized based on rose genomic information" has been added as a special technical feature to claims 1 to 5 and 8, which are independent claims.

2. Explanation
 The written opinion (WO/ISA) states that the invention according to claims 1 to 27 does not share any special technical feature on the basis of Document 1 (JP2011-527183 A), and therefore does not meet the unity of invention requirement.
 In light of this point, the applicant has made an amendment to add the phrase "Vio gene comprising a codon optimized based on rose genomic information" as a special technical feature to each independent claim, based on the description in, for example, paragraph 0014 of the specification of the subject application.
 Thus, the subject application is believed to meet the unity of invention requirement for the present invention as amended.
 In addition, the written opinion states that the present invention lacks novelty and/or inventive step over Documents 1 to 12. However, none of these documents teaches or suggests the "Vio gene comprising a codon optimized based on rose genomic information", and thus the applicant believes that the present invention as amended meets the requirement of novelty and inventive step.
